# EUROPEAN PATENT SPECIFICATION

(11) **EP 3 248 599 B1**
(45) Date of publication and mention of the grant of the patent: **06.07.2022**
(21) Application number: 16740228.8
(22) Date of filing: 20.01.2016
(51) Int. Cl.: A61K 9/107, A61K 31/232, A61K 31/47, A61K 31/505, A61K 9/66, A61K 47/24, A61K 47/26, A61K 47/44, A61P 3/06

(54) **OMEGA-3 FATTY ACID SELF-EMULSIFYING COMPOSITION**
SELBSTEMULGIERENDE OMEGA-3-FETTSÄUREZUSAMMENSETZUNG
COMPOSITION AUTO-ÉMULSIFIANTE D'ACIDES GRAS OMEGA-3

(30) Priority: 21.01.2015 JP 2015009742
(43) Date of publication of application: 29.11.2017
(73) Proprietor: Mochida Pharmaceutical Co., Ltd., Tokyo 160-8515 (JP)
(72) Inventor: ITO, Hiromitsu, Tokyo 160-8515 (JP); FUJII, Hirosato, Tokyo 160-8515 (JP); YAMAGATA, Motoo, Tokyo 160-8515 (JP); TANAKA, Daichi, Tokyo 160-8515 (JP)
(74) Representative: Grünecker Patent- und Rechtsanwälte PartG mbB
(86) International application number: PCT/JP2016/051611
(87) International publication number: WO 2016/117621

(56) References cited:
- EP-A1- 2 433 630
- WO-A1-2010/134614
- WO-A1-2015/008848
- WO-A1-2015/008849
- WO-A2-2012/032414

## Description

### TECHNICAL FIELD

The present invention provides a pharmaceutical composition as defined in claim 1, said composition containing eicosapentaenoic acid ethyl ester as its first medicinal component and pitavastatin, rosuvastatin, or a salt thereof as its second medicinal component.

### BACKGROUND ART

Known ω3 polyunsaturated fatty acids (hereinafter abbreviated as ω3 PUFA) include α-linolenic acid, eicosapentaenoic acid (hereinafter abbreviated as EPA), and docosahexaenoic acid (hereinafter abbreviated as DHA). Since the ω3 PUFA and pharmaceutically acceptable salts and esters thereof (hereinafter abbreviated as ω3 PUFA) have a wide variety of actions such as anti-arteriosclerosis action, platelet aggregation suppressive action, blood lipid lowering action, anti-inflammatory action, carcinostatic action, and central action, they are blended in various food products, and commercially sold in the form of health food, and medical and pharmaceutical products.

Ethyl eicosapentaenoate ester (hereinafter abbreviated as EPA-E) is commercially sold in Japan as an oral therapeutic agent for ameliorating ulcer, pain, and coldness associated with arteriosclerosis obliterans as well as hyperlipidemia (product name Epadel, Mochida Pharmaceutical Co., Ltd.). When EPA-E is orally administered under fasting, increase in plasma EPA concentration is smaller than the case of the oral administration after the meal conceivably because absorption of the EPA-E requires secretion of bile acid and food components as a carrier. Accordingly, Epadel is instructed to be orally administered immediately after the meal (see Non-Patent Literature 1).

In the meanwhile, pitavastatin and rosuvastatin are therapeutic agents for hypercholesterolemia classified as HMG-CoA reductase inhibitors, and these agents play a central role in the treatment of hyperlipidemia together with atorvastatin.

Recently, preparations containing two or more medicinal components have been developed in view of improving medication adherence by patients. However, these preparations have many problems to be obviated including interaction between the medicinal components, solubility, and stability, and development of such preparations is not easy.

A composition containing a ω3 PUFA, a particular statin, and a particular ionic emulsifier has been reported (Patent Literature 1). However, a composition containing EPA-E as the ω3 polyunsaturated fatty acid constituting the main component is not discussed.

An oral liquid pharmaceutical composition containing an ω3 PUFA, a statin drug, a sucrose fatty acid ester, and sorbitan sesquioleate, and a capsule containing such composition have been reported (Patent Literature 2). However, a composition containing EPA-E as the ω3 polyunsaturated fatty acid constituting the main component is not discussed.

With regard to self-emulsifying compositions having a low ethanol content, a self-emulsifying composition comprising an ω3 PUFA, an emulsifier having a hydrophile lipophile balance (hereinafter abbreviated as HLB) of at least 10, lecithin, and a polyhydric alcohol such as propylene glycol or glycerin which has high self-emulsifying property, and high oral absorption property and absorption rate under fasting has been reported (Patent Literature 3).

A therapeutic agent for hyperlipidemia containing a pitavastatin and eicosapentaenoic acid or its ester derivative as its medicinal components has also been reported (Patent Literature 4). However, there has so far been no detailed description for a preparation containing both components.

### CITATION LIST

### Patent Literatures

[Patent Literature 1] WO 2014/095628
[Patent Literature 2] KR 2013-0123564 A
[Patent Literature 3] WO 2010/134614
[Patent Literature 4] JP 5474276 B

### Non-Patent Literature

[Non-Patent Literature 1] Epadel S, Drug Interview Form, Mochida Pharmaceutical Co., Ltd., June, 2012 WO 2012/032414 A2 discloses preconcentrates comprising a fatty acid oil mixture, at least one surfactant, and at least one statin or pharmaceutically acceptable salt, hydrate, solvate, or complex thereof, and uses thereof are disclosed. The preconcentrates may be capable of forming a self-nanoemulsifying drug delivery system, a self-microemulsifying drug delivery system, or self-emulsifying drug delivery systems in an aqueous solution.

### SUMMARY OF INVENTION

### TECHNICAL PROBLEMS

There is a demand for a pharmaceutical composition containing a ω3 PUFA and a statin, wherein administration at a single dose is enough.

There is also a demand for a pharmaceutical composition having a daily dose of the statin incorporated with a daily dose of the ω3 PUFA.

There is also a demand for a transparent pharmaceutical composition having a daily dose of the statin incorporated with a daily dose of the ω3 PUFA.

There is also a demand for a pharmaceutical composition wherein the ω3 PUFA and the statin in the preparation are kept stable.

There is also a demand for a pharmaceutical composition wherein the composition has excellent self-emulsifying property of the ω3 PUFA in the preparation, dispersibility of the composition, and emulsion stability.

There is also a demand for a pharmaceutical composition wherein the ω3 PUFA and the statin in the preparation exhibit excellent releasability in the digestive tract upon administration.

There is also a demand for a pharmaceutical composition wherein the ω3 PUFA and the statin in the preparation exhibit excellent absorption property upon administration.

There is also a demand for a pharmaceutical composition which exhibits a pharmaceutical effect equivalent to the case of combined administration of separate preparations each solely containing its medicinal component although at least one medicinal component is used at a low dose.

There is also a demand for a pharmaceutical composition wherein side effects are smaller compared to the case of combined administration of the preparations each solely containing its medicinal component.

There is also a demand for a pharmaceutical composition whose administration is easier due to the reduced volume compared to the case of combined administration of the preparations each solely containing its medicinal component.

There is also a demand for a pharmaceutical composition with higher medication adherence compared to the case of combined administration of the preparations each solely containing its medicinal component.

There is also a demand for a preparation wherein amounts of ethanol and polyhydric alcohol in the preparation has been reduced.

There is also a demand for a pharmaceutical composition wherein denaturing as represented by cloudiness or separation of the pharmaceutical composition is not recognized even when stored in a low temperature or high temperature environment.

There is also a demand for a pharmaceutical composition wherein softening of a capsule film has been suppressed so that the pharmaceutical composition is not deformed when encapsulated.

Accordingly, an object of the present invention is to provide a pharmaceutical composition wherein at least one of such properties as described above has been improved as well as a preparation having such pharmaceutical composition encapsulated therein.

Another object of the present invention is to provide a pharmaceutical composition wherein at least one of such properties as described above has been improved as well as a method for treating hyperlipidemia by a preparation having such pharmaceutical composition encapsulated therein.

### SOLUTION TO PROBLEMS

In view of the situation as described above, the inventors of the present invention conducted an intensive investigation and found that, in a pharmaceutical composition containing EPA-E, water, an emulsifier containing polyoxyethylene sorbitan fatty acid ester, lecithin, and pitavastatin, rosuvastatin, or a salt thereof, the pitavastatin, rosuvastatin, or a salt thereof is dissolved or uniformly dispersed; and that both the EPA-E and the pitavastatin, rosuvastatin, or a salt thereof are stably retained.

The inventors also found that a pharmaceutical composition which is excellent in at least one of the aspects as described above can be produced, and the present invention was thereby completed.

Accordingly, the present invention provides a pharmaceutical composition comprising, in relation to 100% by weight of a total amount of a self-emulsifying composition:
a) 70 to 90% by weight of eicosapentaenoic acid ethyl ester as a first medicinal component;
b) 0.5 to 6% by weight of water;
c) 1 to 29% by weight of
   (i) polyoxyethylene sorbitan fatty acid ester and
   (ii) polyoxyethylene hydrogenated castor oil and/or polyoxyethylene castor oil

as emulsifiers;
   d) 1 to 25 parts by weight of lecithin in relation to 100 parts by weight of the eicosapentaenoic acid ethyl ester; and
   e) pitavastatin, rosuvastatin, or a salt thereof as a second medicinal component,
wherein
   f) ethanol constitutes up to 4% by weight of the total amount of the self-emulsifying composition, and
   g) polyhydric alcohol constitutes up to 4% by weight of the total amount of the self-emulsifying composition.

In another aspect, the present invention provides an encapsulated pharmaceutical composition obtainable by encapsulating the pharmaceutical composition according to the invention in a hard capsule and/or a soft capsule.

Preferred embodiments are set forth in the subclaims.

A first aspect of the present specification discloses a pharmaceutical composition as described below in items (1-1) to (1-29). The pharmaceutical composition according to a combination of items (1-1) and (1-3), and any pharmaceutical composition according to the remaining items that relates to said pharmaceutical composition is according to the invention. The pharmaceutical compositions according to the remaining items are not according to the invention. The use described below in item (1-23) is also not according to the invention.
(1-1) A pharmaceutical composition comprising, in relation to 100% by weight of a total amount of a self-emulsifying composition:
   a) 70 to 90% by weight of EPA-E as a first medicinal component;
   b) 0.5 to 6% by weight of water;
   c) 1 to 29% by weight of an emulsifier (lecithin is not calculated as the emulsifier defined in the present invention), which is preferably polyoxyethylene sorbitan fatty acid ester;
   d) 1 to 25 parts by weight of lecithin in relation to 100 parts by weight of the EPA-E; and
   e) pitavastatin, rosuvastatin, or a salt thereof as a second medicinal component,
   wherein
   f) ethanol constitutes up to 4% by weight of the total amount of the self-emulsifying composition, and
   g) polyhydric alcohol constitutes up to 4% by weight of the total amount of the self-emulsifying composition.
(1-2) The pharmaceutical composition according to (1-1), wherein the composition contains 0.01 to 1 part by weight of pitavastatin or its salt in relation to 100 parts by weight of the EPA-E, or 0.03 to 5 parts by weight of rosuvastatin or its salt in relation to 100 parts by weight of the EPA-E.
(1-3) The pharmaceutical composition according to (1-1) or (1-2), wherein the emulsifier further comprises polyoxyethylene hydrogenated castor oil and/or polyoxyethylene castor oil.
(1-4) The pharmaceutical composition according to (1-1) to (1-3), wherein the emulsifier further comprises polyoxyethylene castor oil.
(1-5) The pharmaceutical composition according to any one of (1-1) to (1-4), wherein the pitavastatin, rosuvastatin, or a salt thereof is pitavastatin calcium or rosuvastatin calcium.
(1-6) The pharmaceutical composition according to any one of claims (1-1) to (1-5), wherein the lecithin is at least one member selected from the group consisting of soybean lecithin, zymolytic soybean lecithin, hydrogenated soybean lecithin, and egg yolk lecithin.
(1-7) The pharmaceutical composition according to any one of claims (1-1) to (1-6), wherein the lecithin is soybean lecithin.
(1-8) The pharmaceutical composition according to any one of (1-1) to (1-7), wherein the polyoxyethylene sorbitan fatty acid ester comprises polyoxyethylene (20) sorbitan monooleate and/or polyoxyethylene (20) sorbitan trioleate.
(1-9) The pharmaceutical composition according to any one of (1-1) to (1-8), wherein the polyoxyethylene sorbitan fatty acid ester comprises polyoxyethylene (20) sorbitan monooleate.
(1-10) The pharmaceutical composition according to any one of (1-1) to (1-9), wherein the polyoxyethylene castor oil is Polyoxyl 35 castor oil.
(1-11) The pharmaceutical composition according to any one of (1-1) to (1-10), wherein a single dose of the pharmaceutical composition contains 0.5 to 4 g of the EPA-E and 0.3 to 4 mg of the pitavastatin or its salt, or 0.5 to 4 g of the EPA-E and 0.8 to 20 mg of the rosuvastatin or its salt.
(1-12) The pharmaceutical composition according to any one of (1-1) to (1-11), wherein a) to e) are mixed in an arbitrary order.
(1-13) The pharmaceutical composition according to any one of (1-1) to (1-12), wherein the pharmaceutical composition has a transparent appearance when the pharmaceutical composition is allowed to stand.
(1-14) The pharmaceutical composition according to any one of (1-1) to (1-13), wherein the pharmaceutical composition has an appearance without separation or cloudiness when the pharmaceutical composition is allowed to stand.
(1-15) The pharmaceutical composition according to any one of (1-1) to (1-14), wherein the pharmaceutical composition has a transparent appearance when the pharmaceutical composition is stored in an environment of 5°C or 40°C for 12 hours.
(1-16) The pharmaceutical composition according to any one of (1-1) to (1-15), wherein the pharmaceutical composition has an appearance without separation or cloudiness when the pharmaceutical composition is stored in an environment of 5°C or 40°C for 12 hours.
(1-17) The pharmaceutical composition according to any one of (1-1) to (1-16), wherein the pharmaceutical composition is excellent in at least one of self-emulsifying property, dispersibility of the composition, and emulsion stability.
(1-18) The pharmaceutical composition according to any one of (1-1) to (1-17), wherein the pharmaceutical composition emulsifies by itself when 10 µL of the pharmaceutical composition is added dropwise to 5 mL of purified water or a first solution of a dissolution test of Japanese Pharmacopoeia at 37°C.
(1-19) The pharmaceutical composition according to any one of (1-1) to (1-18), wherein the pharmaceutical composition disperses when 10 µL of the pharmaceutical composition is added dropwise to 5 mL of purified water or a first solution of a dissolution test of Japanese Pharmacopoeia at 37°c and stirred.
(1-20) The pharmaceutical composition according to any one of (1-1) to (1-19), wherein the pharmaceutical composition does not experience separation of oil when 10 µL of the pharmaceutical composition is added dropwise to 5 mL of purified water or a first solution of a dissolution test of Japanese Pharmacopoeia at 37°c.
(1-21) The pharmaceutical composition according to any one of (1-1) to (1-20), wherein a mean emulsion droplet diameter upon dispersion of the pharmaceutical composition of the present invention in water is up to 2 µm, or the mean emulsion droplet diameter is up to 1.5 µm, or the mean emulsion droplet diameter is up to 1.0 µm, or the mean emulsion droplet diameter is up to 0.5 µm, or the mean emulsion droplet diameter is up to 0.3 µm.
(1-22) The pharmaceutical composition according to any one of (1-1) to (1-21), wherein, when the pharmaceutical composition according to any one of (1-1) to (1-21) in an amount of 600 mg per dog in terms of EPA-E is orally administered to male beagles under a condition of at least 18 hours of fasting, a maximum plasma EPA concentration (also referred to as a maximum blood EPA concentration) calculated by conducting correction which includes subtracting a plasma EPA concentration before administration is at least 50 µg/mL and/or an area under a curve of a blood EPA concentration at 0 to 2 hours after the administration is at least 30 µg·hr/mL; the maximum plasma EPA concentration is at least 50 µg/mL and/or the area under the curve of the blood EPA concentration at 0 to 2 hours after the administration is at least 50 µg·hr/mL; the maximum plasma EPA concentration is at least 60 µg/mL and/or the area under the curve of the blood EPA concentration at 0 to 2 hours after the administration is at least 60 µg·hr/mL; or the maximum plasma EPA concentration is at least 70 µg/mL and/or the area under the curve of the blood EPA concentration at 0 to 2 hours after the administration is at least 70 µg·hr/mL.
(1-23) Use of the pharmaceutical composition according to any one of (1-1) to (1-21), wherein, when the pharmaceutical composition according to any one of (1-1) to (1-21) in an amount in term of the EPA-E of 1800 mg per person is orally administered to each human before meal, a maximum plasma EPA concentration calculated by conducting correction which includes subtracting a plasma EPA concentration before administration is at least 50 µg/mL and/or a blood EPA concentration 2 hours after the administration is at least 10 µg/mL.
(1-24) The pharmaceutical composition according to any one of (1-1) to (1-21), wherein, when the pharmaceutical composition according to any one of (1-1) to (1-21) in an amount in term of the EPA-E of 1800 mg per person is orally administered to each human before meal, a maximum plasma EPA concentration calculated by conducting correction which includes subtracting a plasma EPA concentration before administration is at least 10 µg/mL and/or an area under a curve of a blood EPA concentration at 0 to 72 hours after the administration is at least 250 µg·hr/mL.
(1-25) The pharmaceutical composition according to any one of (1-1) to (1-24), wherein polyoxyethylene castor oil constitutes up to 120 parts by weight in relation to 100 parts by weight of the polyoxyethylene sorbitan fatty acid ester in the pharmaceutical composition.
(1-26) A pharmaceutical composition comprising, in relation to 100% by weight of a total amount of a self-emulsifying composition:
   a) 70 to 90% by weight of EPA-E as a first medicinal component;
   b) 0.5 to 6% by weight of water;
   c) 1 to 29% by weight of polyoxyethylene sorbitan fatty acid ester as an emulsifier;
   d) 1 to 25 parts by weight of lecithin in relation to 100 parts by weight of the EPA-E; and
   e) pitavastatin, rosuvastatin, or a salt thereof as a second medicinal component,
   wherein
   f) ethanol and/or polyhydric alcohol constitutes up to 4% by weight of the total amount of the self-emulsifying composition.
(1-27) A pharmaceutical composition comprising, in relation to 100% by weight of a total amount of a self-emulsifying composition:
   a) 70 to 90% by weight of EPA-E as a first medicinal component;
   b) 0.5 to 6% by weight of water;
   c) 1 to 29% by weight of polyoxyethylene sorbitan fatty acid ester as an emulsifier;
   d) 1 to 25 parts by weight of lecithin in relation to 100 parts by weight of the EPA-E; and
   e) pitavastatin, rosuvastatin, or a salt thereof as a second medicinal component,
   wherein
   f) ethanol constitutes up to 4% by weight of the total amount of the self-emulsifying composition, and
   g) polyhydric alcohol constitutes up to 4% by weight of the total amount of the self-emulsifying composition.
(1-28) A pharmaceutical composition comprising, in relation to 100% by weight of a total amount of a self-emulsifying composition:
   a) 70 to 90% by weight of EPA-E as a first medicinal component:
   b) 0.5 to 6% by weight of water;
   c) 1 to 29% by weight of polyoxyethylene sorbitan fatty acid ester as an emulsifier;
   d) 1 to 25 parts by weight of lecithin in relation to 100 parts by weight of the EPA-E; and
   e) 0.01 to 1 part by weight of pitavastatin or a salt thereof in relation to 100 parts by weight of the EPA-E, or 0.03 to 5 parts by weight of rosuvastatin or a salt thereof in relation to 100 parts by weight of the EPA-E as a second medicinal component,
   wherein
   f) ethanol constitutes up to 4% by weight of the total amount of the self-emulsifying composition, and
   g) polyhydric alcohol constitutes up to 4% by weight of the total amount of the self-emulsifying composition.
(1-29) The pharmaceutical composition according to (1-28) selected from a pharmaceutical composition having the pitavastatin, rosuvastatin, or a salt thereof dissolved or substantially uniformly dispersed in other components of the pharmaceutical composition, a pharmaceutical composition wherein the pitavastatin, rosuvastatin, or a salt thereof in a form of microcapsules is dispersed in the self-emulsifying composition, a pharmaceutical composition wherein the pitavastatin, rosuvastatin, or a salt thereof is coated on a capsule containing the self-emulsifying composition, and a pharmaceutical composition wherein the pitavastatin, rosuvastatin, or a salt thereof is dissolved or dispersed in a film of a capsule containing the self-emulsifying composition containing the EPA-E.

A second aspect of the present specification discloses an encapsulated pharmaceutical composition as described below in items (2-1) to (2-7). Said encapsulated pharmaceutical composition is according to the invention insofar as it relates to the use of a pharmaceutical composition according to a combination of items (1-1) and (1-3), or any preferred embodiment thereof. All other encapsulated pharmaceutical compositions are not according to the invention.
(2-1) An encapsulated pharmaceutical composition produced by using the pharmaceutical composition according to any one of (1-1) to (1-29) for its content, and encapsulating the content in a hard capsule and/or a soft capsule.
(2-2) The encapsulated pharmaceutical composition according to (2-1) having a high hardness immediately after production.
(2-3) The encapsulated pharmaceutical composition according to (2-1) or (2-2) having a hardness immediately after production of at least 177 N (18 kgf).
(2-4) The encapsulated pharmaceutical composition according to any one of (2-1) to (2-3), wherein, when a preparation is placed in an aluminum package, sealed, and stored at 40°C for 1 week, hardness is not reduced by 59 N (6 kgf) or more compared with hardness before storage.
(2-5) The encapsulated pharmaceutical composition according to any one of (2-1) to (2-4), wherein hardness after placing a preparation in an aluminum package, sealing, and storing at 40°C for 1 week is at least 196 N (20 kgf).
(2-6) The encapsulated pharmaceutical composition according to any one of (2-1) to (2-5), wherein hardness after placing a preparation in an aluminum package, sealing, and storing at 40°C for 1 week is at least 60% of hardness before storage.
(2-7) The pharmaceutical composition according to any one of (2-1) to (2-6) which is at least one member selected from the group consisting of a therapeutic agent for dyslipidemia (hypercholesterolemia, hyper-LDL cholesterolemia, hyper-non-HDL cholesterolemia, hyper-VLDL cholesterolemia, hypo-HDL cholesterolemia, hypertriglyceridemia, hyper-ApoB-emia, hypo-ApoAI-emia, etc.), a therapeutic agent for postprandial hyperglycemia, an antiarteriosclerotic agent, a platelet aggregation suppressive agent, therapeutic agent for peripheral circulatory insufficiency, agent for preventing onset of cardiovascular events, therapeutic agent for inflammatory diseases (nonalcoholic fatty liver disease (hereinafter referred to as NAFLD), non-alcoholic steatohepatitis (hereinafter referred to as NASH), etc.), agents for treating or suppressing the progress of dementia (Alzheimer-type dementia, vascular dementia, mixed-type dementia, etc.), anticancer agent, and therapeutic agent for central diseases (depression, depressive state, obsessive-compulsive disorder, social phobia, panic disorder, etc.).

A third aspect of the present specification discloses a method for producing a pharmaceutical composition as described below in items (3-1) to (3-3). Said method is not according to the invention.
(3-1) A method for producing a pharmaceutical composition comprising: mixing following a) to e) in an arbitrary order in relation to 100% by weight of a total amount of a self-emulsifying composition:
   a) 70 to 90% by weight of EPA-E as a first medicinal component;
   b) 0.5 to 6% by weight of water;
   c) 1 to 29% by weight of polyoxyethylene sorbitan fatty acid ester as an emulsifier;
   d) 1 to 25 parts by weight of lecithin in relation to 100 parts by weight of the EPA-E; and
   e) pitavastatin, rosuvastatin, or a salt thereof as a second medicinal component,
   wherein
   f) ethanol and/or polyhydric alcohol constitutes up to 4% by weight of the total amount of the self-emulsifying composition.
(3-2) The method for producing a pharmaceutical composition according to (3-1), wherein the a), b) and/or c) in the step as described above is heated to at least 70°C and mixed.
   In addition to the third aspect of the present specification, there is also a method for producing a capsule preparation. Said method is not according to the invention.
(3-3) A method for producing a capsule preparation wherein the pharmaceutical composition produced in (3-1) or (3-2) is used for content and this content is subjected to encapsulation; and the thus produced capsule preparation. Preferably, a method for producing a soft capsule preparation wherein the soft capsule preparation is produced by using gelatin as its main component; and the thus produced soft capsule preparation.

A fourth aspect of the present specification discloses a pharmaceutical administered by a particular administration method of the pharmaceutical composition as described below in items (4-1) to (4-6) which is not according to the invention.
(4-1) A preparation for oral administration of the pharmaceutical composition, encapsulated pharmaceutical composition, drug, or veterinary drug according to any one of (1-1) to (1-29) or (2-1) to (2-7) under fasting or before going to bed.
(4-2) A preparation for oral administration of a pharmaceutical composition, an encapsulated pharmaceutical composition, a drug, or a veterinary drug produced by the production method according to (3-1) or (3-2) under fasting or before going to bed.
(4-3) The preparation according to (4-1) or (4-2), wherein the drug is at least one member selected from the group consisting of a therapeutic agent for dyslipidemia (hypercholesterolemia, hyper-LDL cholesterolemia, hyper-non-HDL cholesterolemia, hyper-VLDL cholesterolemia, hypo-HDL cholesterolemia, hypertriglyceridemia, hyper-ApoB-emia, hypo-ApoAI-emia, etc.), a therapeutic agent for postprandial hyperglycemia, an antiarteriosclerotic agent, a platelet aggregation suppressive agent, a therapeutic agent for peripheral circulatory insufficiency, an agent for preventing onset of cardiovascular events, a therapeutic agent for inflammatory diseases (NAFLD, NASH, etc.), an anticancer agent, and an agent for preventing, treating, or preventing progress of central diseases (depression, depressive state, obsessive-compulsive disorder, social phobia, panic disorder, etc.).
(4-4) The preparation according to any one of (4-1) to (4-3), wherein the preparation is administered once a day.
(4-5) A method for administering and/or using the preparation according to any one of (4-1) to (4-4).
(4-6) A method for increasing concentration of the EPA and/or the pitavastatin or rosuvastatin in plasma by orally administering the preparation according to any one of (4-1) to (4-4).

A fifth aspect of the present specification discloses a method for preventing, preventing progress of, or treating at least one disease selected from the group as described below in items (5-1) to (5-4) which is not according to the invention.
(5-1) A method for preventing, preventing progress of, and treating a disease by orally administering at least one pharmaceutical composition, encapsulated pharmaceutical composition, drug, or veterinary drug selected from (1-1) to (1-29), (2-1) to (2-7), (3-1), and (3-2) to patients, wherein the disease is at least one disease selected from the group consisting of dyslipidemia (hypercholesterolemia, hyper-LDL cholesterolemia, hyper-non-HDL cholesterolemia, hyper-VLDL cholesterolemia, hypo-HDL cholesterolemia, hypertriglyceridemia, hyper-ApoB-emia, hypo-ApoAI-emia, etc.), postprandial hyperglycemia, arteriosclerosis, enhanced platelet aggregation, peripheral circulatory insufficiency, onset of cardiovascular events, inflammatory diseases (NAFLD, NASH, etc.), dementia (Alzheimer-type dementia, vascular dementia, mixed-type dementia, etc.), cancer, and central diseases (depression, depressive state, obsessive-compulsive disorder, social phobia, panic disorder, etc.).
(5-2) The method for preventing, preventing progress of, and treating a disease according to (5-1), wherein the disease is dyslipidemia (hypercholesterolemia, hyper-LDL cholesterolemia, hyper-non-HDL cholesterolemia, hyper-VLDL cholesterolemia, hypo-HDL cholesterolemia, hypertriglyceridemia, hyper-ApoB-emia, hypo-ApoAI-emia, etc.).
(5-3) The method according to (5-1) or (5-2), wherein the pharmaceutical composition, the encapsulated pharmaceutical composition, the drug, or the veterinary drug is orally administered under fasting or before going to bed.
(5-4) The method according to any one of (5-1) to (5-3), wherein the pharmaceutical composition, the encapsulated pharmaceutical composition, the drug, or the veterinary drug is administered once a day.

A sixth aspect of the present specification discloses a pharmaceutical composition as described below in items (6-1) to (6-3) which is not according to the invention.
(6-1) A pharmaceutical composition wherein, when the pharmaceutical composition, an encapsulated pharmaceutical composition, a drug, or a veterinary drug containing at least one self-emulsifying composition selected from those produced in (1-1) to (1-29), (2-1) to (2-7), (3-1), and (3-2) as its medicinal component in an amount of 600 mg per dog in terms of EPA-E is orally administered to male beagles under a condition of at least 18 hours of fasting, a maximum plasma EPA concentration calculated by conducting correction which includes subtracting a plasma EPA concentration before administration is at least 50 µg/mL and/or an area under a curve of a blood EPA concentration at 0 to 2 hours after the administration is at least 30 µg·hr/mL; the maximum plasma EPA concentration is at least 50 µg/mL and/or the area under the curve of the blood EPA concentration at 0 to 2 hours after the administration is at least 50 µg·hr/mL; the maximum plasma EPA concentration is at least 60 µg/mL and/or the area under the curve of the blood EPA concentration at 0 to 2 hours after the administration is at least 60 µg·hr/mL; or the maximum plasma EPA concentration is at least 70 µg/mL and/or the area under the curve of the blood EPA concentration at 0 to 2 hours after the administration is at least 70 µg·hr/mL.
(6-2) A pharmaceutical composition wherein, when at least one pharmaceutical composition, encapsulated pharmaceutical composition, or a drug selected from (1-1) to (1-29), (2-1) to (2-7), (3-1), or (3-2) in an amount in term of EPA-E of 1800 mg per person is orally administered to each human before meal, a maximum plasma EPA concentration calculated by conducting correction which includes subtracting a plasma EPA concentration before administration is at least 50 µg/mL and/or a blood EPA concentration 2 hours after the administration is at least 10 µg/mL.
(6-3) A pharmaceutical composition wherein, when at least one pharmaceutical composition, encapsulated pharmaceutical composition, or a drug selected from (1-1) to (1-28), (2-1) to (2-7), (3-1), or (3-2) in an amount in term of EPA-E of 1800 mg per person is orally administered to each human before meal, a maximum plasma EPA concentration calculated by conducting correction which includes subtracting a plasma EPA concentration before administration is at least 10 µg/mL and/or an area under a curve of a blood EPA concentration at 0 to 72 hours after the administration is at least 250 µg·hr/mL.

### ADVANTAGEOUS EFFECTS OF INVENTION

The pharmaceutical composition of the present invention is a pharmaceutical composition containing a daily dose of EPA-E and a daily dose of pitavastatin, rosuvastatin, or a salt thereof, and accordingly, administration at a single daily dose is enough. The pharmaceutical composition of the present invention contains a small amount of water instead of ethanol and polyhydric alcohol in the pharmaceutical composition. Compatibility of the pharmaceutical composition is improved by such composition, and the amount of the emulsifier used can also be reduced, and safety for animals (including human) is thereby improved. In addition, the EPA-E will be included at a higher content, and this enables reduction in the amount of emulsifier used, and compliance is thereby improved.

Inclusion of the water in the pharmaceutical composition also enables a composition without or with a minimized content of the ethanol or the polyhydric alcohol, and the capsule film is prevented from softening, and deformation of the capsule does not occur.

The pharmaceutical composition of the present invention is excellent in at least one of compatibility (appearance), self-emulsifying property, dispersibility of the composition, emulsion stability, and absorbability, and even when administered before the meal or after ingestion of a low-fat diet, is rapidly absorbed to suppress increase in the serum TG after the meal, or when administered before going to bed, prevents essential fatty acid deficiency upon administration of a lipase inhibitor.

The pharmaceutical composition of the present invention is excellent in at least one of solubility, stability in the preparation, releasability in the digestive tract, and absorption from the digestive tract of pitavastatin, rosuvastatin, or a salt thereof.

Further, the composition as described above enables not only the storage at room temperature but also the storage under the conditions of low temperature (for example, 5°C) and high temperature (for example, 40°C) without causing separation or cloudiness of the pharmaceutical composition, namely, with good appearance.

The pharmaceutical composition of the present invention has at least one, preferably at least 2, and more preferably all of the preferable features as described above.

### DESCRIPTION OF EMBODIMENTS

Next, the present invention is described in detail.

The present invention relates to a self-emulsifying pharmaceutical composition as defined in claim 1, said composition containing 70 to 90% by weight of EPA-E as its first medicinal component, 1 to 29% by weight of a particular emulsifier, 1 to 25 parts by weight of lecithin in relation to 100 parts by weight of the EPA-E, pitavastatin, rosuvastatin, or a salt thereof as its second medicinal component, and no ethanol or polyhydric alcohol or the ethanol or polyhydric alcohol at a low concentration; and an encapsulated preparation containing such pharmaceutical composition as its content. The present specification also discloses a drug thereof, its production method, and a method of its use which are not according to the invention.

In the pharmaceutical composition of the present invention, namely, in the pharmaceutical composition containing the EPA-E, the water, the emulsifier containing the polyoxyethylene sorbitan fatty acid ester, the lecithin, and the pitavastatin, rosuvastatin, or a salt thereof, the composition containing the components other than the pitavastatin, rosuvastatin, or a salt thereof is referred to as a self-emulsifying composition. The self-emulsifying composition exhibits good self-emulsifying property. The pharmaceutical composition of the present invention containing such self-emulsifying composition and the pitavastatin, rosuvastatin, or a salt thereof also exhibits good self-emulsifying property.

The eicosapentaenoic acid ethyl ester as used in the present invention is ethyl ester (CAS registry number: 86227-47-6) of eicosapentaenoic acid (CAS registry number: 10417-94-4) belonging to ω3 PUFA, and the eicosapentaenoic acid ethyl ester may be a synthetic, semi-synthetic, or natural eicosapentaenoic acid ethyl ester, or a natural oil containing such eicosapentaenoic acid ethyl ester. Examples of the natural eicosapentaenoic acid ethyl ester include an extract from a natural oil containing eicosapentaenoic acid, a crudely purified natural oil containing eicosapentaenoic acid, and a more highly purified natural oil containing eicosapentaenoic acid, which are produced by methods known in the art. Exemplary semi-synthetic eicosapentaenoic acid ethyl esters include eicosapentaenoic acids produced by microorganisms and such eicosapentaenoic acids or the natural eicosapentaenoic acids which have been subjected to a chemical treatment such as esterification or ester exchange.

The pharmaceutical composition of the present invention preferably has an EPA purity such that the composition has substantially no DHA.

The pharmaceutical composition may also contain a polyunsaturated fatty acid other than the ω3 PUFA such as linoleic acid, γ linolenic acid or dihomo-γ-linolenic acid or a pharmaceutically acceptable salt or ester thereof. However, arachidonic acid or a pharmaceutically acceptable salt or ester thereof is desirably contained in a low amount, preferably less than 2% by weight, more preferably less than 1% by weight, and most preferably, the composition is substantially free from the arachidonic acid or the pharmaceutically acceptable salt or ester thereof.

In the pharmaceutical composition of the present invention, the eicosapentaenoic acid ethyl ester content in relation to 100% by weight of the total amount of the self-emulsifying composition is 70 to 90% by weight, preferably 70 to 86% by weight, more preferably 72 to 85% by weight, and still more preferably 74 to 84% by weight.

Alternatively, in the pharmaceutical composition of the present invention, the eicosapentaenoic acid ethyl ester content in relation to 100% by weight of the total amount of the self-emulsifying composition is 70 to 90% by weight, preferably 70 to 86% by weight, more preferably 70 to 83% by weight, and still more preferably 70 to 80% by weight.

The eicosapentaenoic acid ethyl ester used may be content of a soft capsule preparation containing a high purity EPA-E (at least 96.5% by weight) (product name, Epadel; manufactured by Mochida Pharmaceutical Co., Ltd.) commercially available in Japan as a therapeutic agent for ASO and hyperlipidemia or a soft capsule preparation containing a high purity EPA-E (product name, VASCEPA; Amarin) commercially available in the U.S. as a therapeutic agent for hypertriglyceridemia.

The pitavastatin or a salt thereof as used in the present invention includes pitavastatin (CAS registry number: 147511-69-1), pitavastatin calcium (CAS registry number: 147526-32-7), and pitavastatin sodium (CAS registry number: 574705-92-3), and the preferred is pitavastatin calcium. The pitavastatin or a salt thereof used in the pharmaceutical composition of the present invention can be produced, for example, by the method described in JP 2569746 B. Alternatively, the pitavastatin or a salt thereof used may be a commercially available product. Pitavastatin calcium is sold in Japan as a medical drug (product name, LIVALO Tablet or LIVALO OD Tablet; manufactured by Kowa Company, Ltd.).

The rosuvastatin or a salt thereof as used in the present invention includes rosuvastatin (CAS registry number: 287714-41-4) and rosuvastatin calcium (CAS registry number: 147098-20-2), and the preferred is rosuvastatin calcium. The rosuvastatin or a salt thereof used in the pharmaceutical composition of the present invention can be produced, for example, by the method described in JP 2648897 B. Alternatively, the rosuvastatin or a salt thereof used may be a commercially available product. Rosuvastatin calcium is sold in Japan as a medical drug (product name, CRESTOR Tablet; manufactured by AstraZeneca K.K.).

In the present invention, the "polyoxyethylene sorbitan fatty acid ester" is polyoxyethylene ether of a fatty acid ester wherein some of hydroxy groups of anhydrous sorbitol have been esterified with a fatty acid. Various compounds with different esterifying fatty acids are commercially available, and examples include polyoxyethylene (20) sorbitan monolaurate (NIKKOL TL-10, Polysorbate 20, Tween 20), polyoxyethylene (20) sorbitan monopalmitate (NIKKOL TP-10V, Polysorbate 40, Tween 40), polyoxyethylene (20) sorbitan monostearate (NIKKOL TS-10MV, Polysorbate 60, Tween 60), polyoxyethylene (20) sorbitan tristearate (NIKKOL TS-30V, Polysorbate 65), polyoxyethylene (20) sorbitan monoisostearate (NIKKOL TI-10V), polyoxyethylene (20) sorbitan monooleate (NIKKOL TO-10MV, Polysorbate 80, Tween 80), and polyoxyethylene (20) sorbitan trioleate (NIKKOL TO-30V, Polysorbate 85), and the preferred are polyoxyethylene (20) sorbitan monooleate and polyoxyethylene (20) sorbitan trioleate, and the more preferred is polyoxyethylene (20) sorbitan monooleate.

These may be used alone or in combination of two or more. The term "polyoxyethylene sorbitan fatty acid ester" as used in the present invention includes all of such compounds.

The polyoxyethylene sorbitan fatty acid ester content in the pharmaceutical composition of the present invention is not particularly limited as long as the merits of the present invention are realized. The content is 1 to 29% by weight, preferably 3 to 20% by weight, more preferably 5 to 15% by weight, and most preferably 5 to 9% by weight when the total amount of the self-emulsifying composition is 100% by weight.

Alternatively, the polyoxyethylene sorbitan fatty acid ester content in the pharmaceutical composition of the present invention is preferably 3 to 20% by weight, more preferably 5 to 20% by weight, and most preferably 5 to 15% by weight when the total amount of the self-emulsifying composition is 100% by weight.

In the present invention, the "polyoxyethylene castor oil" is a compound prepared by addition polymerization of ethylene oxide to castor oil. Various compounds with different average degrees of polymerization of ethylene oxide are commercially available, and examples include NIKKOL CO-3 (Nikko Chemicals Co., Ltd.) with an average ethylene oxide mole number of 3, NIKKOL CO-10 (Nikko Chemicals Co., Ltd.) with an average ethylene oxide mole number of 10, EMALEX C-20 (Nihon Emulsion Co., Ltd.) with an average ethylene oxide mole number of 20, EMALEX C-30 (Nihon Emulsion Co., Ltd.) with an average ethylene oxide mole number of 30, Kolliphor EL (BASF) (Polyoxyl 35 castor oil) with an average ethylene oxide mole number of 35, EMALEX C-40 (Nihon Emulsion Co., Ltd.) with an average ethylene oxide mole number of 40, and EMALEX C-50 (Nihon Emulsion Co., Ltd.) with an average ethylene oxide mole number of 50, and the preferred is Kolliphor EL. These may be used alone or in combination of two or more. The term "polyoxyethylene castor oil" as used in the present invention includes all of such compounds unless otherwise noted.

The polyoxyethylene castor oil content in the pharmaceutical composition of the present invention is typically 1 to 20% by weight, preferably 2 to 15% by weight, more preferably 3 to 10% by weight, and most preferably 5 to 9% by weight when the total amount of the self-emulsifying composition is 100% by weight.

Alternatively, the polyoxyethylene castor oil content in the pharmaceutical composition of the present invention is typically 1 to 20% by weight, preferably 2 to 20% by weight, more preferably 3 to 20% by weight, and most preferably 5 to 15% by weight when the total amount of the self-emulsifying composition is 100% by weight.

In addition, the polyoxyethylene castor oil is preferably incorporated in the pharmaceutical composition in the proportion, in relation to 100 parts by weight of the polyoxyethylene sorbitan fatty acid ester, of up to 150 parts by weight, preferably up to 140 parts by weight, more preferably up to 130 parts by weight, still more preferably up to 120 parts by weight, even more preferably up to 110 parts by weight, and most preferably up to 100 parts by weight. The polyoxyethylene sorbitan fatty acid ester and the polyoxyethylene castor oil are preferably incorporated in the pharmaceutical composition so that the content ratio of the polyoxyethylene sorbitan fatty acid ester to the polyoxyethylene castor oil in the pharmaceutical composition is 100 parts by weight : 5 to 150 parts by weight, preferably 100 parts by weight : 10 to 140 parts by weight, more preferably 100 parts by weight : 20 to 130 parts by weight and still more preferably 30 to 120 parts by weight, even more preferably 100 parts by weight : 50 to 110 parts by weight, and most preferably 100 parts by weight : 80 to 120 parts by weight.

In the present invention, the "polyoxyethylene hydrogenated castor oil" is a compound prepared by hydrogenating castor oil with hydrogen, and subjecting the resulting hydrogenated castor oil to addition polymerization with ethylene oxide. Various ethylene oxide compounds having different average degrees of polymerization are commercially available, and examples include polyoxyethylene (20) hydrogenated castor oil (NIKKOL HCO-20, Nikko Chemicals Co., Ltd.), polyoxyethylene (40) hydrogenated castor oil (NIKKOL HCO-40, Nikko Chemicals Co., Ltd.), polyoxyethylene (50) hydrogenated castor oil (NIKKOL HCO-50, Nikko Chemicals Co., Ltd.), polyoxyethylene (60) hydrogenated castor oil (NIKKOL HCO-60, Nikko Chemicals Co., Ltd.), and polyoxyethylene (100) hydrogenated castor oil (NIKKOL HCO-100, Nikko Chemicals Co., Ltd.), and the preferred is, for example, polyoxyethylene (60) hydrogenated castor oil. These may be used alone or in combination of two or more. Unless otherwise noted, the term "polyoxyethylene hydrogenated castor oil" of the present invention includes all of such compounds.

The polyoxyethylene hydrogenated castor oil content in the pharmaceutical composition of the present invention is typically 1 to 20% by weight, preferably 2 to 15% by weight, more preferably 3 to 10% by weight, and most preferably 5 to 9% by weight when the total amount of the self-emulsifying composition is 100% by weight. In addition, the polyoxyethylene hydrogenated castor oil is preferably incorporated in the pharmaceutical composition in the proportion, in relation to 100 parts by weight of the polyoxyethylene sorbitan fatty acid ester, of up to 150 parts by weight, preferably up to 140 parts by weight, more preferably up to 130 parts by weight, still more preferably up to 120 parts by weight, even more preferably up to 110 parts by weight, and most preferably up to 100 parts by weight. The polyoxyethylene sorbitan fatty acid ester and the polyoxyethylene hydrogenated castor oil are suitably incorporated so that the content ratio of the polyoxyethylene sorbitan fatty acid ester to the polyoxyethylene hydrogenated castor oil in the pharmaceutical composition is 100 parts by weight : 5 to 150 parts by weight, preferably 100 parts by weight : 10 to 140 parts by weight, more preferably 100 parts by weight : 20 to 130 parts by weight and still more preferably 100 parts by weight : 30 to 120 parts by weight, even more preferably 100 parts by weight : 50 to 110 parts by weight, and most preferably 100 parts by weight : 80 to 120 parts by weight.

The pharmaceutical composition of the present invention has the characteristic feature that it contains polyoxyethylene sorbitan fatty acid ester and polyoxyethylene castor oil and/or polyoxyethylene hydrogenated castor oil as the emulsifiers. In a preferred embodiment of the present invention, the composition contains polyoxyethylene sorbitan fatty acid ester and polyoxyethylene castor oil as the emulsifiers. The pharmaceutical composition of the present invention may contain polyoxyethylene sorbitan fatty acid ester and an emulsifier other than polyoxyethylene castor oil as the emulsifiers, and the content is up to 20 parts by weight, more preferably up to 10 parts by weight, still more preferably less than 5 parts by weight, and most preferably substantially zero when the total amount of the emulsifier used in the pharmaceutical composition is 100 parts by weight. The emulsifier which may be additionally incorporated is not particularly limited as long as at least one of the requirements as described above can be met, and examples include sorbitan fatty acid ester, glycerin fatty acid ester, polyoxyethylene hydrogenated castor oil, propylene glycol fatty acid ester, saturated polyglycolated glyceride, polyoxyethylene polyoxypropylene glycol, sucrose fatty acid ester, polyethylene glycol fatty acid ester, and tocopherol - polyethylene glycol - succinic acid ester (TPGS) .

The total emulsifier content in the pharmaceutical composition of the present invention is not particularly limited as long as the intended effects of the present invention are realized. However, when the total amount of the self-emulsifying composition is 100% by weight, the total content is 1 to 29% by weight, preferably 3 to 27% by weight, more preferably 5 to 27% by weight, still more preferably 5 to 24% by weight, and most preferably 10 to 20% by weight. Alternatively, the total content is preferably 8 to 27% by weight, and more preferably 10 to 27% by weight. In addition, the total content in relation to 100 parts by weight of the EPA-E is 5 to 45 parts by weight, preferably 10 to 45 parts by weight, more preferably 15 to 35 parts by weight, and most preferably 15 to 25 parts by weight.

The pharmaceutical composition and the pharmaceutical preparation of the present invention contain a small amount of water. Addition of water to a pharmaceutical composition containing a hydrophobic lipid is generally conceived as a loss of compatibility. Presence of water in the composition in itself results in the improved compatibility of the pharmaceutical composition, and the use of the polyhydric alcohol and the ethanol becomes unnecessary. In other words, a product which has a transparent appearance and is free from the problem of separation or cloudiness of the pharmaceutical composition can be produced without using the polyhydric alcohol or the ethanol.

The small amount of water may be added during the preparation of the pharmaceutical composition, and the water in the gelatin capsule film may transfer to the pharmaceutical composition after the encapsulation of the pharmaceutical composition in the capsule. The water is included in the pharmaceutical composition of the present invention as long as the content is within the range defined in the present invention irrespective of its origin.

In addition, the composition free from the polyhydric alcohol and the ethanol does not cause softening and deformation of the capsule after the encapsulation, and such composition is free from the side effects of the ethanol on alcohol intolerance patients upon administration of the composition.

The water is preferably used in an amount of 0.5 to 6% by weight, more preferably 0.5 to 4% by weight, more preferably 0.5 to 3% by weight, and most preferably 1 to 3% by weight when the total amount of the self-emulsifying composition is 100% by weight. Alternatively, the water is preferably used in an amount of at least 0.5% by weight and less than 3% by weight, and more preferably, at least 0.5% by weight and less than 1.5% by weight.

In the present invention, the "lecithin" is a type of glycerophospholipid, and examples include soybean lecithin, zymolytic soybean lecithin, hydrogenated soybean lecithin, soybean phospholipid, purified soybean phospholipid, hydrogenated soybean phospholipid, egg yolk lecithin, egg yolk phopholipid, hydrogenated phospholipid, phospholipid from milk, high purity synthetic phospholipid, unsaturated phospholipid, lysolecithin, phospholipid premix, phosphatidic acid, phosphatidylethanolamine, phosphatidylcholine (purified phosphatidylcholine and purified egg yolk phosphatidylcholine, hydrogenated phosphatidylcholine, polyenephosphatidylcholine, and hydrogenated purified egg yolk phosphatidylcholine), phosphatidylserine, phosphatidylglycerol (purified phosphatidylglycerol and purified egg yolk phosphatidylglycerol, hydrogenated phosphatidylglycerol), phosphatidylinositol, cardiolipin, α-glycerophosphocholine, and purified egg yolk sphingomyelin. The preferred are soybean lecithin, zymolytic soybean lecithin, hydrogenated soybean lecithin, and egg yolk lecithin, and the more preferred is soybean lecithin. These may be used alone or in combination of two or more. The term "lecithin" as used in the present invention includes all of such glycerophospholipids unless otherwise noted. In the present invention, lecithin is not calculated as the emulsifier defined in the present invention.

Various lecithins are commercially available, and exemplary such products include purified soybean lecithin (The Nisshin Oillio Group, Ltd.), purified egg yolk lecithin (Asahi Kasei Pharma Corporation), and egg yolk lecithin PL-100M (Kewpie Corporation). Exemplary soybean lecithins include BASIS LP-20B (Nisshin Oil Mills, Ltd.) and Lipoid S45 and S20 (Lipoid GmbH), and exemplary zymolytic lecithins include BASIS LP-20E (Nisshin Oil Mills, Ltd.) and Phospholipon RLPC20 (Lipoid GmbH). Various such commercially available products may be used in the composition.

The lecithin content in the pharmaceutical composition of the present invention in relation to 100 parts by weight of the EPA-E is preferably 3 to 25 parts by weight, still even more preferably 3 to 20 parts by weight, even still more preferably 3.2 to 17 parts by weight, still further preferably 3.5 to 15 parts by weight, and most preferably 3.7 to 17 parts by weight. Alternatively, the content is preferably 3 to 15 parts by weight, more preferably 3 to 12 parts by weight, and still more preferably 3 to 10 parts by weight. Most preferably, the content is 5 to 10 parts by weight.

The lecithin content in relation to 100% by weight of the total amount of the self-emulsifying composition is preferably 2.1 to 20% by weight, and still more preferably 2.1 to 15% by weight. Alternatively, the content is preferably 1 to 20% by weight, and even more preferably 2 to 15% by weight. Most preferably, the content is 2.1 to 10% by weight.

The lecithin content in relation to 100 parts by weight of the total emulsifier content in the self-emulsifying composition (lecithin is not calculated as the emulsifier defined in the present invention) is preferably 10 to 75 parts by weight, more preferably 11 to 60 parts by weight, still more preferably 20 to 55 parts by weight, and most preferably 25 to 35 parts by weight. Alternatively, the lecithin content in relation to 100 parts by weight of the total emulsifier content in the self-emulsifying composition is preferably 5 to 50 parts by weight, more preferably 6 to 40 parts by weight, still more preferably 7 to 30 parts by weight, and most preferably 8 to 30 parts by weight.

The lecithin content in relation to 100 parts by weight of the total polyoxyethylene sorbitan fatty acid ester content in the self-emulsifying composition is preferably 10 to 150 parts by weight, more preferably 20 to 120 parts by weight, and still more preferably 40 to 90 parts by weight. Most preferably, the content is 50 to 70 parts by weight. Alternatively, the lecithin content in relation to 100 parts by weight of the total polyoxyethylene sorbitan fatty acid ester content in the self-emulsifying composition is preferably 10 to 100 parts by weight, more preferably 15 to 80 parts by weight, and still more preferably 15 to 60 parts by weight. Most preferably, the content is 15 to 40 parts by weight.

In the present invention, the "polyhydric alcohol" is a polyol compound having the structure of a straight chain or cyclic aliphatic hydrocarbon wherein two or more carbon atoms are each substituted with one hydroxy group. Exemplary such polyhydric alcohols include dihydric alcohols such as ethylene glycol, propylene glycol, trimethylene glycol, 1,2-butylene glycol, tetramethylene glycol, 1,3-butylene glycol, 2,3-butylene glycol, and pentamethylene glycol; trihydric alcohols such as glycerin, trimethylolpropane, and 1,2,6-hexanetriol; and polyhydric alcohol polymers such as diethylene glycol, dipropylene glycol triethylene glycol, polyethylene glycol, polypropylene glycol, and polyglycerin, and the preferred is propylene glycol or glycerin. The glycerin also includes concentrated glycerin. The term "polyhydric alcohol" as used in the present invention includes all of such polyol compounds unless otherwise noted.

The polyhydric alcohol content in the pharmaceutical composition of the present invention is such an amount that the capsule is not deformed when the pharmaceutical composition is filled in the capsule. The polyhydric alcohol content in the self-emulsifying composition is not more than 4% by weight when the total composition amount is 100% by weight. The polyhydric alcohol content in the composition is up to 4% by weight, preferably up to 3% by weight, more preferably up to 2% by weight, still more preferably up to 1% by weight, and most preferably 0% by weight.

The ethanol content in the pharmaceutical composition of the present invention is such an amount that change in the quality is not induced during the encapsulation, distribution, or storage, and denaturing of the capsule content is not induced. Also, the ethanol content should not exceed the daily experientially allowable medical dose. The ethanol content in the composition is not more than 4% by weight when the total self-emulsifying composition amount is 100% by weight. The ethanol content in the composition is up to 4% by weight, preferably up to 3% by weight, more preferably up to 2% by weight, still more preferably up to 1% by weight, and most preferably 0% by weight.

When the ethanol and the polyhydric alcohol are included in the pharmaceutical composition, the ethanol and the polyhydric alcohol are preferably included in the self-emulsifying composition in a total amount of not more than 4% by weight when the total amount of the self-emulsifying composition is 100% by weight. In the preferred embodiment, the composition does not substantially contain the ethanol or the polyhydric alcohol. The total amount of the ethanol and the polyhydric alcohol in the composition is preferably up to 4% by weight, more preferably up to 3% by weight, even more preferably up to 2% by weight, still more preferably up to 1% by weight, and most preferably 0% by weight.

The preferable ethanol concentration may be appropriately determined based on the EPA-E content and the daily dose of the pharmaceutical composition of the present invention. When the pharmaceutical composition of the present invention is orally administered at a daily dose of 1800 mg in terms of the EPA-E and a preparation containing, for example, 75% by weight of the EPA-E is prepared, the ethanol dose will not exceed 3.26 mg which is the daily maximum dose described in the "Japanese Pharmaceutical Excipients Directory" when the ethanol concentration is up to 0.135% by weight.

The pharmaceutical composition of the present invention may be filled in a capsule. The capsule selected may be a hard capsule or a soft capsule, and preferably, the capsule used is a soft capsule. The soft capsule is not particularly limited for its shape, and preferably, the soft capsule is a rotary die type soft capsule or a seamless capsule.

In the soft capsule of the present invention, the capsule film is not necessarily limited for its composition, and exemplary main ingredients include gelatin, carrageenan, pectin, pullulan, sodium alginate, starch, hypromellose, hydroxypropyl cellulose, and other known ingredients. The preferred is gelatin, and the type of gelatin used is not particularly limited. Exemplary gelatins include acid-treated gelatin, alkali-treated gelatin, amphoteric gelatin, chemically modified gelatin, and other known gelatins, which may be used alone or in combination of two or more. The gelatin used is preferably an acid-treated gelatin or alkali-treated gelatin. The source of the gelatin is not necessarily limited, and the gelatin used may be the one from cattle bone, cattle skin, pig bone, pig skin, fish scale, or fish skin, and preferably, the one from cattle bone, cattle skin, pig bone, or pig skin.

The "gelatin" used may be the one normally used in the production of a soft capsule, for example, medical gelatin (gelatin and purified gelatin) defined in The Japanese Pharmacopoeia 16th edition. The gelatin may also be a combination of two or more types, and the capsule film may also contain other components such as a plasticizing agent.

The "plasticizing agent" added to the capsule film may be the one normally used in the production of a soft capsule, with preferred examples including a polyhydric alcohol such as glycerin (for example, concentrated glycerin), ethylene glycol, polyethylene glycol, propylene glycol, or polypropylene glycol, and a sugar alcohol such as sorbitol, mannitol, or xylitol. These plasticizing agents may be used in combination of two or more. Particularly preferred are glycerin and sorbitol. Also preferred is a combination of glycerin and sorbitol, and in this case, the glycerin and the sorbitol may be used at a weight ratio in the range of 1:5 to 5:1, and more preferably 1:3 to 3:1.

In the soft capsule preparation, and in particular, in the seamless capsule of the present invention, the capsule film solution preferably contains the gelatin and the plasticizing agent at a weight ratio in the range of 10:1 to 1:10, and more preferably 10:1 to 1:1.

The weight ratio of the capsule film solution to the capsule content is typically 10:1 to 1:10, and preferably 3:1 to 1:10.

If desired, the capsule film may also contain various additives commonly used in the capsule film. Exemplary such additives include amino acids, citric acid, glycerin, sorbitol, trehalose, and other plasticizing agents, antiseptic, dye, titanium oxide, and other colorants, and organic acids.

The composition for the capsule film may be prepared by dissolving the gelatin, the plasticizing agent, and the optional additives in water at room temperature or at an elevated temperature.

Preferably, the capsule having the pharmaceutical composition of the present invention encapsulated as its content has high hardness immediately after the production, and this hardness is preferably maintained during the storage. Loss of the hardness is unfavorable in view of the quality because the loss of the hardness does not only result in the deformation but also in the brittleness and cracking of the capsule and bleeding of the content. Softening of the capsule can be detected by measuring the hardness with a common hardness tester.

The capsule of the present invention has a hardness immediately after the production of at least 177 N (18 kgf), preferably at least 196 N (20 kgf), and more preferably at least 216 N (22 kgf). It is desirable that, when the preparation is stored in a tightly sealed aluminum package at 40°C for 1 week, the hardness of the capsule not substantially decrease, or not decrease by 59 N (6 kgf) or more compared with the hardness immediately after the production. The capsule has a hardness after the storage at 40°C for 1 week of at least 98 N (10 kgf), preferably at least 147 N (15 kgf), and more preferably at least 196 N (20 kgf).

When the hardness immediately after the production is 100%, the hardness maintained after the storage in a tightly sealed aluminum package at 40°C for 1 week is not less than 60%, preferably not less than 70%, more preferably not less than 80%, still more preferably not less than 85%, and most preferably not less than 90% of the hardness immediately after the production.

The dose and dosage period of the EPA-E used in the pharmaceutical composition of the present invention are those sufficient for realizing the intended action, and they can be adequately adjusted depending on the administration route, frequency of administration per day, seriousness of the symptoms, body weight, age, and other factors.

More specifically, 0.1 to 10 g/day, preferably 0.2 to 8 g/day, more preferably 0.3 to 5 g/day, and still more preferably 0.3 to 4 g/day of the EPA-E may be administered in a single dose to 3 divided doses but the total amount may be optionally administered in a single dose to several divided doses. The preferred is a single dose/day. In the case of the administration in a single dose/day, soft capsule preparations each containing 0.5 g or 1 g of EPA-E may be adequately combined, and 1 to 10 capsules, preferably 1 to 8 capsules, more preferably 1 to 6 capsules, still more preferably 1 to 4 capsules, and even more preferably 1 to 3 capsules may be administered. In other words, a soft capsule preparation containing 1 g of EPA-E and a soft capsule preparation containing 0.5 g of EPA-E may be adequately combined to enable the administration of 0.5 g/dose, 1.5 g/ dose, 2.5 g/ dose, 3.5 g/dose, 4.5 g/dose or 5.5 g/dose.

Alternatively, a required number of soft capsules, granular capsules, or seamless capsules each containing, for example, 10 mg to 300 mg, preferably 10 mg to 100 mg, more preferably 10 to 75 mg, and still more preferably 25 to 50 mg of EPA-E may be administered.

Since absorption of the EPA-E is affected by the meal, the administration is preferably conducted during or after the meal, and more preferably, immediately after the meal (within 30 minutes after the meal). In the case of the pharmaceutical composition of the present invention which exhibits excellent EPA-E absorption even in fasting condition, the intended effects of the present invention can be realized if the composition is administered at a timing other than during or after the meal or immediately after the meal, namely, for example, under fasting (at least 8 hours, and preferably at least 10 hours after the last meal), before the meal, immediately before the meal, in between the meals, or before going to bed; if the composition is administered to a patient suffering from reduced absorption ability of the intestinal tract (elderly, patient with an intestinal disease, patient after the intestinal surgery, terminal cancer patient, during the administration of lipase inhibitor), or if the dosage is reduced.

The dose and dosage period of the pitavastatin, rosuvastatin, or a salt thereof used in the pharmaceutical composition of the present invention are those sufficient for realizing the intended action, and they can be adequately adjusted depending on the administration route, frequency of administration per day, seriousness of the symptoms, body weight, age, and other factors.

The amount of the pitavastatin, rosuvastatin, or a salt thereof incorporated in the single dose of the pharmaceutical composition of the present invention is not particularly limited. However, the amount incorporated in the single dose is preferably 1/10 to twice the normal daily dose of the medicinal component, more preferably 1/3 to normal daily dose, and still more preferably the normal daily dose. In the case of the combination where synergetic effects or improvement in the mutual absorption property by simultaneous incorporation is expected, the amount may be adequately reduced, and more specifically, the pitavastatin or a salt thereof is preferably incorporated in an amount of 0.1 mg to 8 mg, more preferably 0.3 mg to 4 mg, and still more preferably 1 to 4 mg. Alternatively, in the case when synergetic effects or improvement in the mutual absorption property by simultaneous incorporation is expected, the pitavastatin or a salt thereof may be incorporated in an amount of 0.03 mg to 2.7 mg, more preferably 0.1 mg to 1.3 mg, and still more preferably 0.3 to 1.3 mg; and the rosuvastatin or a salt thereof may be incorporated in an amount of 0.25 mg to 40 mg, more preferably 0.8 mg to 20 mg, and still more preferably 2.5 to 20 mg. Alternatively, in the case when synergetic effects or improvement in the mutual absorption property by simultaneous incorporation is expected, the rosuvastatin or a salt thereof may be incorporated in an amount of preferably 0.08 mg to 13 mg, more preferably 0.3 mg to 7 mg, and still more preferably 0.8 to 7 mg.

The pitavastatin, rosuvastatin, or a salt thereof used in the pharmaceutical composition of the present invention is not particularly limited for its amount of incorporation. The pitavastatin or a salt thereof is preferably incorporated in an amount of 0.01 to 1 part by weight, more preferably 0.02 to 0.8 parts by weight, and still more preferably 0.03 to 0.7 parts by weight in relation to 100 parts by weight of the EPA-E. The rosuvastatin or a salt thereof is preferably incorporated in an amount of 0.03 to 5 parts by weight, more preferably 0.05 to 4 parts by weight, and still more preferably 0.08 to 3.5 parts by weight in relation to 100 parts by weight of the EPA-E. In the case of the combination where synergetic effects or improvement in the absorbance by simultaneous incorporation is expected, the amount may be adequately reduced.

Amount of the EPA-E and the pitavastatin or a salt thereof in single dose of the pharmaceutical composition of the present invention is preferably 0.1 to 5 g of the and 0.03 to 8 mg of the pitavastatin or a salt thereof, more preferably 0.5 to 4 g of the EPA-E and 0.1 to 4 mg of the pitavastatin or a salt thereof, and 0.5 to 3 g of the EPA-E and 0.3 to 4 mg of the pitavastatin or a salt thereof are included.

Exemplary combinations include 2 g of the EPA-E and 4 mg of the pitavastatin or a salt thereof, 2 g of the EPA-E and 2 mg of the pitavastatin or a salt thereof, 2 g of the EPA-E and 1 mg of the pitavastatin or a salt thereof, 1 g of the EPA-E and 4 mg of the pitavastatin or a salt thereof, 1 g of the EPA-E and 2 mg of the pitavastatin or a salt thereof, and 1 g of the EPA-E and 1 mg of the pitavastatin or a salt thereof.

The amount of the EPA-E and the rosuvastatin or a salt thereof in a single dose of the pharmaceutical composition of the present invention is preferably such that 0.1 to 5 g of the EPA-E and 0.08 to 40 mg of the rosuvastatin or a salt thereof are included, more preferably 0.5 to 4 g of the EPA-E and 0.3 to 20 mg of the rosuvastatin or a salt thereof are included, and still more preferably 0.5 to 3 g of the EPA-E and 0.8 to 20 mg of the rosuvastatin or a salt thereof are included.

Exemplary combinations include 2 g of the EPA-E and 40 mg of the rosuvastatin or a salt thereof, 2 g of the EPA-E and 20 mg of the rosuvastatin or a salt thereof, 2 g of the EPA-E and 10 mg of the rosuvastatin or a salt thereof, 2 g of the EPA-E and 5 mg of the rosuvastatin or a salt thereof, 2 g of the EPA-E and 2.5 mg of the rosuvastatin or a salt thereof, 1 g of the EPA-E and 40 mg of the rosuvastatin or a salt thereof, 1 g of the EPA-E and 20 mg of the rosuvastatin or a salt thereof, 1 g of the EPA-E and 10 mg of the rosuvastatin or a salt thereof, 1 g of the EPA-E and 5 mg of the rosuvastatin or a salt thereof, and 1 g of the EPA-E and 2.5 mg of the rosuvastatin or a salt thereof.

The amount of the pitavastatin, rosuvastatin, or a salt thereof in relation to the total amount of the pharmaceutical composition of the present invention is, for example, 0.01 to 2% by weight, preferably 0.02 to 1.5% by weight, and more preferably 0.04 to 1.3% by weight in terms of the pitavastatin or a salt thereof; and for example, 0.03 to 10% by weight, preferably 0.05 to 7.5% by weight, more preferably 0.1 to 6.5% by weight in terms of the rosuvastatin or a salt thereof. The content, however, is not limited to such range.

The pharmaceutical composition of the present invention is preferably the one wherein the EPA-E and the pitavastatin, rosuvastatin, or a salt thereof in the pharmaceutical composition is rapidly released and absorbed in the digestive tract at a rate capable of realizing its pharmacological action upon administration of the pharmaceutical composition.

In addition, an adequate carrier or medium, a colorant, a flavor, and optionally, a vegetable oil and an additive such as non-toxic organic solvent or non-toxic solubilizing agent, emulsifier, suspending agent (for example, Tween 80 and gum arabic solution), isotonic agent, pH adjusting agent, stabilizer, corrective, flavoring agent, preservative, antioxidant, or absorption promoter commonly used in the art may be adequately combined with the present composition to prepare an appropriate pharmaceutical preparation.

More specifically, since the eicosapentaenoic acid is highly unsaturated, an effective amount of an oil-soluble antioxidant, for example, at least one member selected from butylated hydroxytoluene, butylated hydroxyanisole, propyl gallate, propyl gallate, pharmaceutically acceptable quinone, astaxanthin, and α-tocopherol is preferably incorporated in the composition as an antioxidant.

With regard to the pharmaceutical composition of the present invention, the method used for producing a preparation by incorporating the EPA-E and the pitavastatin, rosuvastatin, or a salt thereof may be any known method commonly used for producing the preparation of a ω3 PUFA and a statin. Exemplary methods include, for example, a method wherein the pitavastatin, rosuvastatin, or a salt thereof is dissolved or substantially uniformly dispersed in the self-emulsifying composition containing the EPA-E according to the method described in JP 2008-533029 A; a method wherein the pitavastatin, rosuvastatin, or a salt thereof is incorporated in microcapsules and the microcapsules are dispersed in the self-emulsifying composition containing the EPA-E according to the method described in JP 4976302 B; a method wherein the pitavastatin, rosuvastatin, or a salt thereof is coated on a capsule containing the self-emulsifying composition containing the EPA-E according to the method described in JP 5628480 B; and a method wherein the pitavastatin, rosuvastatin, or a salt thereof is dissolved or dispersed in the capsule film of a capsule containing the self-emulsifying composition containing the EPA-E according to the method described in WO 2012/2464. Of these, the preferred is the method wherein the pitavastatin, rosuvastatin, or a salt thereof is dissolved or substantially uniformly dispersed in the self-emulsifying composition containing the EPA-E, and the more preferred is the method wherein the pitavastatin, rosuvastatin, or a salt thereof is dissolved in the self-emulsifying composition containing the EPA-E.

Preferably, the pharmaceutical composition of the present invention has good appearance, self-emulsifying property, dispersibility of the composition, emulsion stability, absorption property, and storage stability of the medicinal components and the preparation. The appearance of the pharmaceutical composition is such that the composition is not separated, clouded, solidified, or precipitated, but is transparent. The composition having poor appearance may be pharmaceutically unsuitable, and such composition may be insufficient in the required performance such as self-emulsifying property.

With regard to the storage temperature, the pharmaceutical composition and the capsulated preparation of the composition are preferably transparent in appearance at both low temperature and high temperature in view of the possibility of its use in a cold district or a hot environment.

In the case of the pharmaceutical composition having good self-emulsifying property, good dispersibility of the composition, and high emulsion stability, the composition rapidly disperses upon contact with water to form a microemulsion having an adequate emulsion droplet diameter. The absorption property of an oil such as EPA-E is related to the size of the emulsion droplet diameter, and the degree of absorption upon administration to an animal can be estimated by measuring the emulsion droplet diameter.

In the present invention, the "mean emulsion droplet diameter" is the value of volume mean diameter among droplets of the emulsified composition as measured by using a particle size analyzer (for example, Nanotrac manufactured by Nikkiso Co., Ltd.) with water being used for the dispersion medium according to a standard measurement method (for example, set zero time of 30 seconds, measurement time of 30 seconds, average of three measurements). The mean emulsion droplet diameter when the pharmaceutical composition of the present invention is dispersed in water is not particularly limited as long as it is up to 2 µm, and the product has good emulsion dispersibility, good emulsion stability, or good absorbability. The mean emulsion droplet diameter is typically up to 1.5 µm, more preferably up to 1.0 µm, still more preferably up to 0.5 µm, and most preferably up to 0.3 µm.

In order to realize pharmacological actions of the EPA-E and the pitavastatin, rosuvastatin, or a salt thereof, the pharmaceutical composition of the present invention preferably has at least one merit selected from good appearance, excellent self-emulsifying property, high dispersibility of the composition, high emulsion stability, high storage stability (including the stability at normal temperature, low temperature, and high temperature), high absorbability, and in particular, high absorbability and absorption rate upon fasting, no change in the absorbability between before and after the meal, medicine-taking convenience for patients, reduced side effects, and high medication compliance.

The pharmaceutical composition of the present invention is well adapted for use as a therapeutic agent for treating various diseases of animals, and in particular, mammals such as a therapeutic agent for dyslipidemia (hypercholesterolemia, hyper-LDL cholesterolemia, hyper-non-HDL cholesterolemia, hyper-VLDL cholesterolemia, hypo-HDL cholesterolemia, hypertriglyceridemia, hyper-ApoB-emia, hypo-ApoAI-emia, etc.), a therapeutic agent for postprandial hypertriglyceridemia, an antiarteriosclerotic agent, a platelet aggregation suppressant, a therapeutic agent for peripheral circulatory insufficiency, an agent for preventing onset of cardiovascular events, a therapeutic agent for inflammatory diseases (NAFLD, NASH, etc.), an agent for treating or suppressing the progress of dementia (Alzheimer-type dementia, vascular dementia, mixed-type dementia, etc.), an anticancer agent, and a therapeutic agent for central diseases (depression, depressive state, obsessive-compulsive disorder, social phobia, panic disorder, etc.). In the treatment of such diseases, the pharmaceutical composition of the present invention may be preferably administered once a day or in 2 or 3 divided doses per day although the daily administration frequency is not particularly limited. It is more preferred to administer once or twice a day, and most preferably once a day.

Of the diseases as mentioned above, the pharmaceutical composition of the present invention is particularly effective in improving, treating or preventing recurrence of dyslipidemia and postprandial hypertriglyceridemia and in preventing progress to metabolic syndrome, cardio- and cerebrovascular events, and ulcer and gangrene at a limb distal end. Exemplary mammals include human, domestic animals such as cattle, horse, and pig, and companion animals such as dog, cat, rabbit, rat, and mouse, and the preferred is human. More specifically, the pharmaceutical composition of the present invention is expected to show ameliorating or therapeutic effects for dyslipidemia and postprandial hypertriglyceridemia in patients with dyslipidemia suffering from increase in the blood lipid, exhibiting insulin resistance or suffering from increase in the blood pressure, such as metabolic syndrome patients.

### Examples

Next, the present invention is described in further detail by referring to Examples and Comparative Examples.

### Reference Example 1

0.06 g of water, 0.36 g of polyoxyethylene (20) sorbitan oleate ester, 0.36 g of Polyoxyl 35 castor oil, 0.22 g of soybean lecithin, and 4.0 g of EPA-E were weighed, sealed in a container, and heated to about 70°C with mixing to prepare a self-emulsifying composition. The resulting self-emulsifying composition was purged with nitrogen, sealed in a container, and stored at room temperature until the evaluation was conducted.

### Reference Example 2

0.1 g of water, 0.29 g of polyoxyethylene (20) sorbitan oleate ester, 0.29 g of Polyoxyl 35 castor oil, 0.32 g of soybean lecithin, and 4.0 g of EPA-E were weighed, sealed in a container, heated to about 70°C with mixing to prepare a self-emulsifying composition. The resulting self-emulsifying composition was purged with nitrogen, sealed in a container, and stored at room temperature until the evaluation was conducted.

### Example 1 and Example 2

(1) 2.12 g of water, 18 g of polyoxyethylene (20) sorbitan oleate ester, 18 g of Polyoxyl 35 castor oil, 11 g of soybean lecithin, and 204.6 g of EPA-E were weighed, sealed in a container, heated to about 70°C with mixing to prepare a self-emulsifying composition.
(2) 6.3 g of the self-emulsifying composition of (1) was weighed, and after adding 10 mg of pitavastatin calcium in the case of Example 1 or 40 mg of pitavastatin calcium in the case of Example 2, the mixture was heated to 50°C, stirred, and subjected to ultrasonication to prepare each pharmaceutical composition. Formulation of the pharmaceutical composition is shown in Table 1. The resulting pharmaceutical composition was purged with nitrogen, sealed in a container, and stored at room temperature until the evaluation was conducted.

### Examples 3 to 8

The procedure of Example 1 was repeated except that each component was weighed and mixed to the compositional ratio shown in Table 1 to prepare each pharmaceutical composition. The resulting pharmaceutical composition was purged with nitrogen, sealed in a container, and stored at room temperature until the evaluation was conducted.

The pharmaceutical compositions and the self-emulsifying compositions of Reference Examples prepared by the procedures as described above are each encapsulated in a soft capsule containing gelatin as its main component.

### Test Example 1 <Evaluation of appearance>

After producing the pharmaceutical compositions and the self-emulsifying compositions of Reference Examples by the production methods as described above, the compositions were allowed to stand, and after about 1 hour, their appearance was evaluated. The composition was evaluated "transparent" when the composition was homogeneous due to the good compatibility. The composition was evaluated "separated" when the separation was observed and "cloudy" when opacity was observed.

The test results of Examples are shown in Table 1.

### Test Example 2 <Evaluation of self-emulsifying property>

The pharmaceutical compositions and the self-emulsifying compositions of Reference Examples produced by the production methods as described above were evaluated for their self-emulsifying property by adding 10 µL of each pharmaceutical composition dropwise to 5 mL of purified water or first solution in the dissolution test of Japanese Pharmacopoeia at 37°C in the test tube. The composition which spontaneously emulsified just by the dropwise addition was evaluated "good", and the case which did not become an emulsion just by the dropwise addition was evaluated "poor". The composition was then lightly stirred under consistent condition, and the properties were evaluated. With regard to the dispersibility of the composition, the composition was evaluated "good" when dispersed and "poor" when the composition was partly left undispersed as a mass. With regard to the emulsion stability, the composition was evaluated "good" when no oil separation was observed and "poor" when oil separation was observed. It is to be noted that the pharmaceutical compositions which were not evaluated as "transparent" in the evaluation of the appearance were not evaluated.

The test results of Examples are shown in Table 1.

### Test Example 3 <Evaluation of emulsion droplet diameter>

The mean emulsion droplet diameter (volume mean diameter) of the emulsified material is evaluated by using about 1.5 mL of the emulsified composition obtained in Test Example 2 using a particle size analyzer (Nanotrac, manufactured by Nikkiso Co., Ltd.) with water being used for the dispersion medium. With regard to the self-emulsifying composition of Reference Example 1, the mean emulsion droplet diameter was 0.27 µm in the test using purified water at 37°C and 0.22 µm in the test using the first solution in the dissolution test of Japanese Pharmacopoeia at 37°C. The pharmaceutical compositions of Example 1 to 8 exhibit similar behavior.

### Test Example 4 <Evaluation of appearance after storage under severe conditions>

The pharmaceutical compositions which were evaluated "transparent" in Test Example 1 were allowed to stand and stored overnight (about 12 hours) at 5°C or 40°C before their appearance was evaluated. When the composition was homogeneous due to the good compatibility, the composition was evaluated "transparent". The composition was evaluated "separated" when the separation was observed and "cloudy" when opacity was observed.

The test results of Examples are shown in Table 1.

### Test Example 5 <Pharmacokinetics in beagles>

The pharmaceutical compositions, the self-emulsifying compositions, or the capsules produced in Examples and Reference Examples are each orally administered to 6 male beagles (at the age of 2 to 6 years with the body weight of 8 to 13 kg, 3 Marshall beagles and 3 Nosan beagles) under fasting conditions, and blood EPA concentration is monitored. The test animals are fasted for 18 hours or more before the administration, and each animal is administered with the composition in an amount corresponding to 600 mg of the EPA-E. Blood is collected before the administration, and 0.5, 1, 1.5, 2, 3, 4, 6, 8, and 24 hours after the administration, and plasma is separated and treated to measure plasma EPA concentration by LC/MS/MS (the method wherein the sample is separated by liquid chromatography, and then, by mass spectroscopy for measurement). The control group is administered with the EPA-E stock solution encapsulated in a capsule.

For Reference Example 1, the maximum plasma concentration (Cmax) calculated from the test results was 128.7 µg/mL, the area under the blood concentration curve at 0 to 2 hours (AUC₀₋₂) also calculated from the test results was 97.8 µg·hr/mL, and the area under the blood concentration curve at 0 to 24 hours (AUC₀₋₂₄) also calculated from the test results was 1036.3 µg·hr/mL. It is to be noted that, in the calculation of each parameter, the value is corrected by subtracting the plasma EPA concentration before the administration from each blood concentration. The pharmaceutical compositions of Examples 1 to 8 exhibit similar behavior.

### Test Example 6 <Appearance of capsule>

Each of the soft capsules obtained in Examples and Reference Examples is visually inspected for the color and shape of the capsule and the properties of the capsule content after completing the filling and the drying.

The capsules with change in the "color", distortion, or depression in the "shape", and cloudiness, or separation in the "properties of the capsule content" are evaluated "poor", and the capsules without such problems are evaluated "normal".

The appearance of the capsule of Reference Example 1 was normal. The capsules encapsulated with the pharmaceutical compositions of Examples 1 to 8 exhibit similar behavior.

In the Table, below, "-" means that the corresponding component was not added or that the corresponding item was not measured.

**[Table 1]**

| Component name | | Example 1 | Example 2 | Example 3 | Example 4 | Example 5 | Example 6 | Example 7 | Example 8 |
|---|---|---|---|---|---|---|---|---|---|
| Ethyl eicosapentaenoate | | 80.5 | 80.1 | 74.9 | 79.9 | 79.9 | 82.9 | 74.3 | 82.7 |
| Purified water | | 0.8 | 0.8 | 1.1 | 1.2 | 2.0 | 2.0 | 1.1 | 2.0 |
| Polyoxyethylene (20) sorbitan oleate ester | | 7.1 | 7.1 | 10.8 | 7.2 | - | 8.0 | 10.7 | 8.0 |
| Polyoxyethylene sorbitan trioleate | | - | - | - | - | 5.8 | - | - | - |
| Polyoxyl 35 castor oil | | 7.1 | 7.1 | 10.8 | 7.2 | 5.8 | - | 10.7 | - |
| Polyoxyethylene hydrogenated castor oil | | - | - | - | - | - | 4.0 | - | 4.0 |
| Soybean lecithin | | 4.3 | 4.3 | 2.4 | 4.4 | 6.4 | 3.0 | 2.4 | 3.0 |
| Pitavastatin calcium | | 0.16 | 0.63 | 0.16 | 0.16 | 0.16 | 0.16 | - | - |
| Rosuvastatin calcium | | - | - | - | - | - | - | 0.8 | 0.4 |
| Total | | 100.0 | 100.0 | 100.2 | 100.1 | 100.1 | 100.1 | 100.0 | 100.1 |
| Test Example 1 | Appearance | Transparent | Cloudy | Transparent | Transparent | Transparent | Transparent | Transparent | Transparent |
| Test Example 2 | Self-emulsifying property | Good | - | Good | Good | Good | Good | Good | Good |
| | Dispersibility of the composition | Good | - | Good | Good | Good | Good | Good | Good |
| | Emulsion stability | Good | - | Good | Good | Good | Good | Good | Good |
| Test Example 4 | When stored at 5°C | Transparent | - | Transparent | Transparent | Transparent | Transparent | Transparent | Transparent |
| Appearance | When stored at 40°C | Transparent | - | Transparent | Transparent | Transparent | Transparent | Transparent | Transparent |

The pharmaceutical composition of Example 1 had the composition including the polyoxyethylene sorbitan fatty acid ester and the polyoxyethylene castor oil as emulsifiers as well as the lecithin and the water within particular ranges, and in this Example, the pitavastatin calcium completely dissolved and the pharmaceutical composition had good appearance as well as good self-emulsifying property and good appearance after storage under severe conditions. The composition also exhibits good absorption in the pharmacokinetics test using beagles.

When 100 mg of atorvastatin calcium was used instead of 10 mg of the pitavastatin calcium to examine according to the method of Example 1, the atorvastatin calcium did not dissolve and the good appearance was not realized.

In Example 2, 10 mg of the pitavastatin calcium used in Example 1 was replaced with 40 mg of the pitavastatin calcium for examination. The pitavastatin calcium was substantially uniformly dispersed while entirely transparent appearance was not obtained. Preparation by encapsulation is possible.

The pharmaceutical composition of Example 2 also has good self-emulsifying property, and further has good appearance after storage under severe conditions, and the composition also exhibits good absorption in the pharmacokinetics test using beagles.

Examples 3 and 4 are variations where the ratio of the components which were the same as those incorporated in Example 1 had been changed. In all pharmaceutical compositions, the pitavastatin calcium completely dissolved, and the pharmaceutical composition had good appearance as well as good self-emulsifying property and good appearance after storage under severe conditions. The composition also exhibits good absorption in the pharmacokinetics test using beagles.

In Example 5, the composition was prepared by using polyoxyethylene (20) sorbitan trioleate instead of the polyoxyethylene (20) sorbitan oleate ester of Example 1, and changing the incorporation ratio of the components including other components. The pitavastatin calcium completely dissolved, and the pharmaceutical composition had good appearance as well as good self-emulsifying property and good appearance after storage under severe conditions. The composition also exhibits good absorption in the pharmacokinetics test using beagles.

In Example 6, the composition was prepared by using HCO-60 which is polyoxyethylene hydrogenated castor oil instead of the Polyoxyl 35 castor oil of Example 1, and changing the incorporation ratio of the components including other components. The pitavastatin calcium completely dissolved, and the pharmaceutical composition had good appearance as well as good self-emulsifying property and good appearance after storage under severe conditions. The composition also exhibits good absorption in the pharmacokinetics test using beagles.

In Example 7, the composition was prepared by using the same components and similar incorporation ratio as those of Example 3 except for the use of the rosuvastatin calcium instead of the pitavastatin calcium. The rosuvastatin calcium completely dissolved, and the pharmaceutical composition had good appearance as well as good self-emulsifying property and good appearance after storage under severe conditions. The composition also exhibits good absorption in the pharmacokinetics test using beagles.

In Example 8, the composition was prepared by using the same components and similar incorporation ratio as those of Example 6 except for the use of the rosuvastatin calcium instead of the pitavastatin calcium. The rosuvastatin calcium completely dissolved, and the pharmaceutical composition had good appearance as well as good self-emulsifying property and good appearance after storage under severe conditions. The composition also exhibits good absorption in the pharmacokinetics test using beagles.

Reference Example 1 provides the self-emulsifying composition not containing the pitavastatin calcium or the rosuvastatin calcium. The composition of Reference Example 1, however, exhibited good appearance, good self-emulsifying property, and also, good appearance after the storage under severe conditions. The composition also exhibited good absorption for the EPA-E in the pharmacokinetics test using beagles.

### Test Example 7 <Hardness of capsule>

Each soft gelatin capsule filled with 375 mg of the self-emulsifying composition of Reference Example 2 (300 mg in terms of EPA-E) was produced by a rotary method. The self-emulsifying capsule preparation prepared by the method of the present invention exhibited no deformation of the capsule film.

The capsule preparation of Reference Example 2 was evaluated for the hardness. The preparation was stored at 40°C and a relative humidity of 75% for 1, 2, and 4 weeks and its hardness was similarly measured.

The initial hardness (N (kgf)) and hardness after storing for 1, 2, and 4 weeks at 40°C of the preparation of Reference Example 2 were 283 (28.9), 250 (25.5), 240 (24.5), and 269 (27.4), respectively. The term "initial" as used herein means that the capsule produced was stored at room temperature until its evaluation. It is to be noted that the capsule is not affected by humidity since the preparation was stored at 40°C after sealing in an aluminum package.

The hardness of the capsule as described herein is the one which can be confirmed by measurement using a common hardness meter.

The preparation produced by encapsulating the self-emulsifying composition of Reference Example 2 has a hardness of at least 196 N (20 kgf) in the initial stage, and the hardness hardly changed when stored in sealed condition at 40°C for 1 to 4 weeks. The capsule preparations having the pharmaceutical compositions of Examples 1 to 6 exhibit similar behavior. In the meanwhile, the capsule preparation containing, for example, approximately 8% of propylene glycol (a polyhydric alcohol) exhibits low hardness from the initial stage, and it lost the hardness with lapse of time.

### Test Example 8-1 <Pharmacokinetics in human (single dose test, amount administered: 1800 mg>

The capsule preparation of Reference Example 1 was orally administered to 12 human subjects (20 to 40 year old healthy adult males having a body weight of 55.0 to 77.0 kg and BMI of at least 18.5 and less than 25.0) under fasting conditions, and blood EPA concentration was monitored. To each human subject, the self-emulsifying composition in an amount in terms of the EPA-E of 1800 mg was orally administered in a single dose in the morning under fasting by using 200 ml of water. After the administration, blood was collected at 0.5, 1, 1.5, 2, 3, 4, 5, 6, 7, 8, 9, 10, 12, 15, 18, 24, 48, and 72 hours after the administration. The collected blood samples were cooled in an ice bath immediately after the blood collection and centrifuged at 2000 x g and at 4°C for 10 minutes, and the separated plasma was cryopreserved at a temperature of -20°C or less. The concentration of the EPA in the resulting plasma was measured by LC/MS/MS (a method wherein a sample is separated by liquid chromatography, and then by mass spectroscopy for measurement).

The capsule preparation of Reference Example 1 was also orally administered immediately after the meal and the measurement was conducted by repeating the procedure as described above.

As a control group, EPA-E stock solution (high purity EPA-E (at least 96.5% by weight) not containing the emulsifier, the same amount in terms of the EPA-E as the self-emulsifying composition of Reference Example 1) encapsulated in capsules was orally administered to 12 human subjects (20 to 40 year old healthy adult males having a body weight of 55.0 to 77.0 kg and BMI of at least 18.5 and less than 25.0) under fasting conditions, and the measurement was conducted by repeating the procedure as described above.

Table 2 shows maximum plasma concentration (Cmax), plasma concentration at 24 hours after the administration (C₂₄), area under the curve of the blood concentration at 0 to 72 hours (AUC₀₋₇₂), time required for reaching the maximum plasma concentration (Tmax), and elimination half-life in plasma (t_{1/2}) calculated from the test results. It is to be noted that, in the calculation of each parameter, the value is corrected by subtracting the plasma EPA concentration before the administration from each blood concentration.

### Test Example 8-2 <Pharmacokinetics in human (single dose test, amount administered: 3600 mg)>

The test was conducted by repeating the procedure of Test Example 8-1 except that the amount of the EPA-E to be administered was changed to 3600 mg. The test was conducted for 6 human cases. Table 3 shows maximum plasma concentration (Cmax), plasma concentration at 24 hours after the administration (C₂₄), area under the curve of the blood concentration at 0 to 72 hours (AUC₀₋₇₂), time required for reaching the maximum plasma concentration (Tmax), and elimination half-life in plasma (t_{1/2}) calculated from the test results. It is to be noted that, in the calculation of each parameter, the value is corrected by subtracting the plasma EPA concentration before the administration from each blood concentration.

**[Table 2]**

| Test Example 8-1 | Capsule preparation of Reference Example 1 (Amount administered, 1800 mg) | Capsule preparation of Reference Example 1 (Amoun t administered, 1800 mg) | Control group EPA-E stock solution |
|---|---|---|---|
| Meal | No | Yes | No |
| Cmax (µg/mL) | 65.1 | 111.3 | 46 |
| C24hr (µg/mL) | 19.5 | 28.6 | 2.4 |
| AUC0-72hr (µg·hr/mL) | 1266.0 | 1932.1 | 113.1 |
| Tmax (hr) | 5.2 | 3.3 | 10.8 |
| t1/2 (hr) | 31.2 | 42.6 | 71.7 |

**[Table 3]**

| Test Example 8-2 | Capsule preparation of Reference Example 1 (Amount administered, 3600 mg) | Capsule preparation of Reference Example 1 (Amoun t administered, 3600 mg) | Control group EPA-E stock solution |
|---|---|---|---|
| Meal | No | Yes | No |
| Cmax (µg/mL) | 174.2 | 184.5 | 3.6 |
| C24hr (µg/mL) | 36.4 | 37.7 | 1.2 |
| AUC0-72hr (µg·hr/mL) | 2845.5 | 2615.9 | 113.7 |
| Tmax (hr) | 5.2 | 4.3 | 21.8 |
| t1/2 (hr) | 58.7 | 42.4 | 22.8 |

It is to be noted that the preparations produced by encapsulating the pharmaceutical compositions of Examples 1 to 8 also exhibit good EPA absorption.

### INDUSTRIAL APPLICABILITY

The pharmaceutical composition of the present invention is a pharmaceutical composition containing a daily dose of the EPA-E and a daily dose of the pitavastatin, rosuvastatin, or a salt thereof, wherein administration at a single daily dose is enough. The pharmaceutical composition of the present invention is excellent in at least one of the compatibility (appearance), self-emulsifying property, dispersibility of the composition, emulsion stability, and absorbability, and, even if taken before the meal, the EPA-E is rapidly absorbed to suppress increase of serum TG after the meal. Alternatively, the pharmaceutical composition of the present invention is excellent in at least one of solubility of the pitavastatin, rosuvastatin, or a salt thereof, stability in the preparation, releasability in the digestive tract, and absorption from the digestive tract.

The polyhydric alcohol content in the pharmaceutical composition of the present invention is zero or low, and therefore, the composition is free from the problem of softening and deformation of the capsule during the distribution or storage caused by the polyhydric alcohol. In other words, the pharmaceutical composition of the present invention is associated with reduced risk of quality change.

The pharmaceutical composition of the present invention retains its quality as a pharmaceutical product even when stored in a cold or hot area since the composition does not become cloudy or separated even if stored in low or high temperature environment.

## Claims

1. A pharmaceutical composition comprising, in relation to 100% by weight of a total amount of a self-emulsifying composition:
a) 70 to 90% by weight of eicosapentaenoic acid ethyl ester as a first medicinal component;
b) 0.5 to 6% by weight of water;
c) 1 to 29% by weight of
(i) polyoxyethylene sorbitan fatty acid ester and
(ii) polyoxyethylene hydrogenated castor oil and/or polyoxyethylene castor oil as emulsifiers;
d) 1 to 25 parts by weight of lecithin in relation to 100 parts by weight of the eicosapentaenoic acid ethyl ester; and
e) pitavastatin, rosuvastatin, or a salt thereof as a second medicinal component,
wherein
f) ethanol constitutes up to 4% by weight of the total amount of the self-emulsifying composition, and
g) polyhydric alcohol constitutes up to 4% by weight of the total amount of the self-emulsifying composition.

2. The pharmaceutical composition according to claim 1, wherein the composition contains 0.01 to 1 part by weight of pitavastatin or its salt in relation to 100 parts by weight of the eicosapentaenoic acid ethyl ester, or 0.03 to 5 parts by weight of rosuvastatin or its salt in relation to 100 parts by weight of the eicosapentaenoic acid ethyl ester.

3. The pharmaceutical composition according to any one of claims 1or 2, wherein the emulsifiers comprise the polyoxyethylene sorbitan fatty acid ester and polyoxyethylene castor oil.

4. The pharmaceutical composition according to any one of claims 1 to 3, wherein the pitavastatin, rosuvastatin, or a salt thereof is pitavastatin calcium or rosuvastatin calcium.

5. The pharmaceutical composition according to any one of claims 1 to 4, wherein the lecithin is soybean lecithin.

6. The pharmaceutical composition according to any one of claims 1 to 5, wherein the polyoxyethylene sorbitan fatty acid ester is polyoxyethylene (20) sorbitan monooleate.

7. The pharmaceutical composition according to any one of claims 1 to 6, wherein the polyoxyethylene castor oil is Polyoxyl 35 castor oil.

8. The pharmaceutical composition according to any one of claims 1 to 7, wherein a single dose of the pharmaceutical composition contains 0.5 to 4 g of the eicosapentaenoic acid ethyl ester and 0.3 to 4 mg of the pitavastatin or its salt, or 0.5 to 4 g of the eicosapentaenoic acid ethyl ester and 0.8 to 20 mg of the rosuvastatin or its salt.

9. The pharmaceutical composition according to any one of claims 1 to 8,
wherein
the composition comprises, in relation to 100% by weight of the total amount of the self-emulsifying composition:
a) 70 to 90% by weight of the eicosapentaenoic acid ethyl ester as the first medicinal component;
b) 0.5 to 6% by weight of the water;
c) 5 to 24% by weight of the polyoxyethylene sorbitan fatty acid ester and polyoxyethylene castor oil as emulsifiers;
d) 1 to 25 parts by weight of the lecithin in relation to 100 parts by weight of the eicosapentaenoic acid ethyl ester; and
e) 0.01 to 1 part by weight of the pitavastatin or its salt in relation to 100 parts by weight of the eicosapentaenoic acid ethyl ester, or 0.03 to 5 parts by weight of the rosuvastatin or its salt in relation to 100 parts by weight of the eicosapentaenoic acid ethyl ester as the second medicinal component, and
wherein
f) the polyoxyethylene castor oil is included in an amount of up to 120 parts by weight in relation to 100 parts by weight of the polyoxyethylene sorbitan fatty acid ester,
g) the ethanol constitutes up to 4% by weight of the total amount of the self-emulsifying composition, and
h) the polyhydric alcohol constitutes up to 4% by weight of the total amount of the self-emulsifying composition.

10. An encapsulated pharmaceutical composition obtainable by encapsulating the pharmaceutical composition according to any one of claims 1 to 9 in a hard capsule and/or a soft capsule.

11. The encapsulated pharmaceutical composition according to claim 10, wherein a capsule film of the soft capsule contains gelatin.

## Patentansprüche

1. Pharmazeutische Zusammensetzung, umfassend, bezogen auf 100 Gew.-% der Gesamtmenge einer selbstemulgierenden Zusammensetzung
a) 70 bis 90 Gew.-% Eicosapentaensäureethylester als erste medizinische Komponente;
b) 0,5 bis 6 Gew.-% Wasser;
c) 1 bis 29 Gew.-%
(i) Polyoxyethylensorbitanfettsäureester und
(ii) Polyoxyethylen-hydriertes Rizinusöl und/oder Polyoxyethylenrizinusöl als Emulgatoren;
d) 1 bis 25 Gewichtsteile Lecithin, bezogen auf 100 Gewichtsteile des Eicosapentaensäureethylesters; und
e) Pitavastatin, Rosuvastatin oder ein Salz davon als zweite medizinische Komponente, wobei
f) Ethanol bis zu 4 Gew.-% der Gesamtmenge der selbstemulgierenden Zusammensetzung ausmacht, und
g) mehrwertiger Alkohol bis zu 4 Gew.-% der Gesamtmenge der selbstemulgierenden Zusammensetzung ausmacht.

2. Die pharmazeutische Zusammensetzung nach Anspruch 1, wobei die Zusammensetzung 0,01 bis 1 Gewichtsteil Pitavastatin oder dessen Salz, bezogen auf 100 Gewichtsteile Eicosapentaensäureethylester, oder 0,03 bis 5 Gewichtsteile Rosuvastatin oder dessen Salz, bezogen auf 100 Gewichtsteile Eicosapentaensäureethylester, enthält.

3. Die pharmazeutische Zusammensetzung nach einem der Ansprüche 1 oder 2, wobei die Emulgatoren den Polyoxyethylensorbitanfettsäureester und Polyoxyethylenrizinusöl umfassen.

4. Die pharmazeutische Zusammensetzung nach einem der Ansprüche 1 bis 3, wobei das Pitavastatin, Rosuvastatin oder ein Salz davon Calcium-Pitavastatin oder Calcium-Rosuvastatin ist.

5. Die pharmazeutische Zusammensetzung nach einem der Ansprüche 1 bis 4, wobei das Lecithin Sojabohnenlecithin ist.

6. Die pharmazeutische Zusammensetzung nach einem der Ansprüche 1 bis 5, wobei der Polyoxyethylensorbitanfettsäureester Polyoxyethylen-(20)-sorbitanmonooleat ist.

7. Die pharmazeutische Zusammensetzung nach einem der Ansprüche 1 bis 6, wobei das Polyoxyethylenrizinusöl Polyoxyl-35-Rizinusöl ist.

8. Die pharmazeutische Zusammensetzung nach einem der Ansprüche 1 bis 7, wobei eine Einzeldosis der pharmazeutischen Zusammensetzung 0,5 bis 4 g Eicosapentaensäureethylester und 0,3 bis 4 mg Pitavastatin oder dessen Salz, oder 0,5 bis 4 g Eicosapentaensäureethylester und 0,8 bis 20 mg Rosuvastatin oder dessen Salz enthält.

9. Die pharmazeutische Zusammensetzung nach einem der Ansprüche 1 bis 8, wobei die Zusammensetzung, bezogen auf 100 Gew.-% der Gesamtmenge der selbstemulgierenden Zusammensetzung, umfasst:
a) 70 bis 90 Gew.-% Eicosapentaensäureethylester als erste medizinische Komponente;
b) 0,5 bis 6 Gew.-% Wasser;
c) 5 bis 24 Gew.-% Polyoxyethylensorbitanfettsäureester und Polyoxyethylenrizinusöl als Emulgatoren;
d) 1 bis 25 Gewichtsteile Lecithin, bezogen auf 100 Gewichtsteile Eicosapentaensäureethylester; und
e) 0,01 bis 1 Gewichtsteil Pitavastatin oder dessen Salz, bezogen auf 100 Gewichtsteile Eicosapentaensäureethylester, oder 0,03 bis 5 Gewichtsteile Rosuvastatin oder dessen Salz, bezogen auf 100 Gewichtsteile Eicosapentaensäureethylester, als zweite medizinische Komponente, und
wobei
f) Polyoxyethylenrizinusöl in einer Menge von bis zu 120 Gewichtsteilen, bezogen auf 100 Gewichtsteile Polyoxyethylensorbitanfettsäureester, enthalten ist,
g) Ethanol bis zu 4 Gew.-% der Gesamtmenge der selbstemulgierenden Zusammensetzung ausmacht, und
h) mehrwertiger Alkohol bis zu 4 Gew.-% der Gesamtmenge der selbstemulgierenden Zusammensetzung ausmacht.

10. Eingekapselte pharmazeutische Zusammensetzung, erhältlich durch Einkapseln der pharmazeutischen Zusammensetzung nach einem der Ansprüche 1 bis 9 in eine Hartkapsel und/oder eine Weichkapsel.

11. Die eingekapselte pharmazeutische Zusammensetzung nach Anspruch 10, wobei eine Kapselfolie der Weichkapsel Gelatine enthält.

## Revendications

1. Composition pharmaceutique comprenant, en relation à 100 % en poids d'une quantité totale d'une composition auto-émulsifiante:
a) de 70 à 90 % en poids d'ester éthylique d'acide éicosapentaénoïque comme premier constituant médicinal ;
b) de 0,5 à 6 % en poids d'eau ;
c) de 1 à 29 % en poids
(i) d'ester d'acide gras de polyoxyéthylène sorbitan et
(ii) d'huile de ricin hydrogénée de polyoxyéthylène et/ou d'huile de ricin de polyoxyéthylène comme agents émulsifiants ;
d) de 1 à 25 parties en poids de lécithine en relation à 100 parties en poids de l'ester éthylique d'acide éicosapentaénoïque ; et
e) de la pitavastatine, rosuvastatine, ou son sel comme second constituant médicinal,
f) l'éthanol constituant jusqu'à 4 % en poids de la quantité totale de la composition auto-émulsifiante, et
g) l'alcool polyhydrique constituant jusqu'à 4 % en poids de la quantité totale de la composition auto-émulsifiante.

2. Composition pharmaceutique selon la revendication 1, la composition contenant de 0,01 à 1 partie en poids de pitavastatine ou son sel en relation à 100 parties en poids de l'ester éthylique d'acide éicosapentaénoïque, ou de 0,03 à 5 parties en poids de rosuvastatine ou son sel en relation à 100 parties en poids de l'ester éthylique d'acide éicosapentaénoïque.

3. Composition pharmaceutique selon l'une quelconque des revendications 1 ou 2, les agents émulsifiants comprenant l'ester d'acide gras de polyoxyéthylène sorbitan et l'huile de ricin de polyoxyéthylène.

4. Composition pharmaceutique selon l'une quelconque des revendications 1 à 3, la pitavastatine, rosuvastatine, ou son sel étant la pitavastatine calcique ou la rosuvastatine calcique.

5. Composition pharmaceutique selon l'une quelconque des revendications 1 à 4, la lécithine étant la lécithine de soja.

6. Composition pharmaceutique selon l'une quelconque des revendications 1 à 5, l'ester d'acide gras de polyoxyéthylène sorbitan étant le monooléate de polyoxyéthylène (20) sorbitan.

7. Composition pharmaceutique selon l'une quelconque des revendications 1 à 6, l'huile de ricin de polyoxyéthylène étant l'huile de ricin polyoxyl 35.

8. Composition pharmaceutique selon l'une quelconque des revendications 1 à 7, une dose unique de la composition pharmaceutique contenant de 0,5 à 4 g d'ester éthylique d'acide éicosapentaénoïque et de 0,3 à 4 mg de la pitavastatine ou son sel, ou de 0,5 à 4 g de l'ester éthylique d'acide éicosapentaénoïque et de 0,8 à 20 mg de la rosuvastatine ou son sel.

9. Composition pharmaceutique selon l'une quelconque des revendications 1 à 8,
la composition comprenant, en relation à 100 % en poids de la quantité totale de la composition auto-émulsifiante :
a) de 70 à 90 % en poids de l'ester éthylique d'acide éicosapentaénoïque comme premier constituant médicinal ;
b) de 0,5 à 6 % en poids de l'eau ;
c) de 5 à 24 % en poids de l'ester d'acide gras de polyoxyéthylène sorbitan et d'huile de ricin de polyoxyéthylène comme agents émulsifiants ;
d) de 1 à 25 parties en poids de la lécithine en relation à 100 parties en poids de l'ester éthylique d'acide éicosapentaénoïque ; et
e) de 0,01 à 1 partie en poids de la pitavastatine ou son sel en relation à 100 parties en poids de l'ester éthylique d'acide éicosapentaénoïque, ou de 0,03 à 5 parties en poids de la rosuvastatine ou son sel en relation à 100 parties en poids de l'ester éthylique d'acide éicosapentaénoïque comme second constituant médicinal, et
f) l'huile de ricin de polyoxyéthylène étant comprise en une quantité allant jusqu'à 120 parties en poids en relation à 100 parties en poids de l'ester d'acide gras de polyoxyéthylène sorbitan,
g) l'éthanol constituant jusqu'à 4 % en poids de la quantité totale de la composition auto-émulsifiante, et
h) l'alcool polyhydrique constituant jusqu'à 4 % en poids de la quantité totale de la composition auto-émulsifiante.

10. Composition pharmaceutique encapsulée pouvant être obtenue par encapsulation de la composition pharmaceutique selon l'une quelconque des revendications 1 à 9 dans une capsule dure et/ou une capsule molle.

11. Composition pharmaceutique encapsulée selon la revendication 10, un film de capsule de la capsule molle contenant de la gélatine.
